# FASCICULE DE BREVET EUROPEEN

(11) **EP 3 187 223 B1**
(45) Date de publication et mention de la délivrance du brevet: **19.09.2018**
(21) Numéro de dépôt: 16202327.9
(22) Date de dépôt: 06.12.2016
(51) Int. Cl.: A61N 1/36, A61N 1/39

(54) **DISPOSITIF MÉDICAL ACTIF DE NEUROSTIMULATION ÉLECTRIQUE, AVEC CONTRÔLE AUTOMATIQUE DE LA COMPENSATION DE CHARGE**
AKTIVE MEDIZINISCHE VORRICHTUNG ZUR ELEKTRISCHEN NEUROSTIMULATION MIT AUTOMATISCHER KONTROLLE DER LADUNGSKOMPENSATION
ACTIVE MEDICAL DEVICE FOR ELECTRIC NERVE STIMULATION, WITH AUTOMATIC CONTROL OF LOAD COMPENSATION

(30) Priorité: 11.12.2015 FR 1562184
(43) Date de publication de la demande: 05.07.2017
(73) Titulaire: Sorin CRM SAS, 92140 Clamart Cedex (FR)
(72) Inventeur: BONNET, Jean-Luc, 91300 MASSY (FR); DUCHEMIN LAPORTE, Laure, 91420 MORANGIS (FR); FOLLENIUS, Arnaud, 75015 PARIS (FR)
(74) Mandataire: Grünecker Patent- und Rechtsanwälte PartG mbB

(56) Documents cités:
- EP-A1- 2 926 863
- US-A1- 2009 048 643
- US-A1- 2010 114 198

## Description

L'invention concerne les dispositifs médicaux actifs.

Elle concerne plus précisément les implants permettant de délivrer des thérapies dites FES (*Functional Electrical Stimulation*), consistant à appliquer à des fins thérapeutiques sur des organes une stimulation sous forme d'impulsions électriques répétées.

L'invention concerne plus particulièrement les implants permettant de délivrer des thérapies de stimulation de tissus biologiques. L'invention porte plus précisément sur la stimulation du système nerveux (ci-après désignée de façon générale "neurostimulation"), notamment mais de façon non limitative de stimulation du nerf vague, technique dite "VNS" (*Vagus Nerve Stimulation*), au moyen d'un dispositif comprenant une sonde pourvue d'une électrode implantée sur le nerf vague ou à proximité de celui-ci, et un générateur délivrant des impulsions électriques VNS sur cette électrode.

Le EP 2 926 863 A1 (Sorin CRM) décrit un tel générateur VNS de stimulation du nerf vague.

Cette application n'est toutefois pas limitative, et l'invention est applicable à d'autres cas où une stimulation de tissus biologiques nécessite la délivrance d'impulsions de compensation.

La stimulation du système nerveux est une approche thérapeutique reconnue ou en cours d'évaluation à l'égard de très nombreux troubles tels que l'épilepsie, la dépression profonde, la douleur, l'insuffisance cardiaque, l'apnée du sommeil, l'obésité, etc. La VNS a démontré des effets positifs dans des études précliniques dans le cadre de l'insuffisance cardiaque, où elle agit sur le système nerveux autonomique et de façon secondaire sur les fonctions cardiovasculaires, induisant une diminution du rythme cardiaque et une augmentation de la fraction d'éjection du ventricule gauche, ce qui peut notamment contribuer à réduire l'évolution d'un remodelage cardiaque susceptible de mener à un état d'insuffisance cardiaque aggravée.

Par son action sur l'équilibre sympathovagal (SVB) du patient, la neurostimulation a également un effet général sur le système vasculaire, avec modulation de la vasoconstriction par modification des diamètres des artères et de la résistance périphérique se traduisant par une vasodilatation générale du système vasculaire.

Les impulsions de neurostimulation peuvent être délivrées de façon synchrone, ou non, avec le rythme cardiaque ou tout autre paramètre physiologique, auquel cas le dispositif comprend des moyens de recueil d'au moins un paramètre physiologique, typiquement des ondes de dépolarisation du myocarde, qui peuvent être mesurées par recueil d'un ECG par une électrode subcutanée, d'un EGM par une électrode implantée sur ou dans le myocarde, ou d'un signal *far-field* recueilli entre le boitier et une électrode placée en dehors du coeur, notamment un pôle de l'électrode de neurostimulation placée sur ou à proximité d'une structure nerveuse.

Les impulsions de neurostimulation étant des impulsions de courant, lorsque l'on stimule un tissu physiologique, l'interface entre l'électrode et le tissu doit rester globalement équilibrée en termes de charge électrique.

Dans le cas d'impulsions en courant constant, la charge Q est définie comme le produit du courant I (en ampères ou milliampères (mA)) par la durée PW de l'impulsion (en secondes ou millisecondes) : Q = I x PW, et s'exprime donc en coulombs, ou plus généralement en microcoulombs (µC) en neurostimulation.

Comme la délivrance de l'impulsion de neurostimulation proprement dite (ci-après "phase de stimulation") va produire, du fait du passage du courant, une création et une accumulation d'une charge au niveau du site de stimulation, cette charge devra être compensée ou annulée par une charge contraire (par circulation d'un courant dans le sens inverse de celui de la phase de stimulation), de manière à maintenir la neutralité électrique globale du tissu stimulé.

Le US2010/0114198 A1 décrit une générateur d'impulsions de stimulation incorporant un circuit assurant de façon automatique une telle compensation de charge.

La charge contraire de compensation (ci-après "phase compensatrice") peut intervenir :
- sous forme passive, par une décharge spontanée dans la bioimpédance formée par les tissus au niveau du site de stimulation, cette décharge (ci-après "décharge passive") ayant lieu postérieurement à l'application d'une impulsion de stimulation isolée, ou d'une salve d'impulsions de stimulation successives ; et/ou
- sous forme active, par une charge résultant d'une impulsion de courant générée par le stimulateur et appliquée au tissu avant ("précharge") ou après ("postcharge") une ou plusieurs impulsions de stimulation.

On appellera "train impulsionnel multiphasique" une telle combinaison de phases temporelles, comprenant i) une phase de stimulation et ii) une phase compensatrice comprenant au moins une précharge/postcharge active ou une décharge passive finale.

Dans la suite, on parlera simplement d'"impulsion de précharge" pour désigner un type d'impulsion compensatrice contrôlée, mais ce terme ne préjuge pas un type particulier de séquence multiphasique d'impulsions, la phase compensatrice contrôlée pouvant être générée non seulement avant, mais également après une phase de stimulation, que celle-ci soit formée d'une impulsion de stimulation isolée ou bien d'une salve d'impulsions de stimulation se succédant à une cadence élevée.

Par ailleurs, différents profils multiphasiques combinant impulsion de précharge et impulsion de stimulation peuvent être envisagés, par exemple avec une impulsion de précharge associée à chaque impulsion de stimulation, ou une impulsion de précharge associée à plusieurs impulsions successives de stimulation, etc., étant précisé que l'invention sera applicable à tout type de profil de stimulation combinant impulsion de précharge (ou de postcharge), impulsion(s) de stimulation et impulsion de décharge passive. L'invention est applicable à tout type de stimulation multiphasique mettant en oeuvre ces différentes phases temporelles de précharge ou postcharge, stimulation et décharge passive, notamment la neurostimulation. Dans la suite du document on fera référence à la précharge, mais l'invention peut être appliquée similairement à une postcharge.

Dans tous les cas, la phase de précharge comprend l'application d'une impulsion de courant de sens contraire de celui de l'impulsion de stimulation, et d'amplitude et de durée contrôlées, afin de produire une charge totale -Q égale mais inversée par rapport à celle Q de la stimulation.

Pour éviter que la précharge ne produise des effets physiologiques, l'amplitude de l'impulsion de précharge est ajustée à un niveau beaucoup plus faible que celui d'une impulsion de stimulation, sa durée étant allongée pour que la quantité de charge correspondante (égale au produit de l'intensité par la durée de l'impulsion) soit du même ordre de grandeur que la charge de stimulation à compenser. Par exemple, une impulsion de stimulation de 3 mA/0,5 ms sera compensée par une impulsion de précharge de 0,5 mA/3 ms.

L'objectif visé par un stimulateur produisant de tels trains impulsionnels multiphasiques est d'obtenir les effets physiologiques attendus - par exemple une réduction du rythme cardiaque, une modification contrôlée de l'équilibre sympathovagal, etc. - en respectant un équilibre global des charges électriques à la fin du train impulsionnel. Cependant, seule la stimulation doit produire un effet physiologique, les phases compensatrices (précharge, postcharge, décharge passive) ne doivent pas être effectives physiologiquement.

Le problème principal, dans ce cas, est de s'assurer que les phases compensatrices (précharge(s) et décharge passive) ne produisent pas d'effets physiologiques indésirables.

Or les nerfs, en particulier le nerf vague qui est souvent la cible d'une thérapie de neurostimulation, sont composés d'un très grand nombre de fibres nerveuses de types différents (type A, B, C notamment pour le nerf vague), chaque type de fibre ayant ses propres caractéristiques de seuil d'activation et de vitesse de propagation de l'influx nerveux. Ainsi, les fibres les plus grosses ont un seuil d'activation bas et une vitesse de propagation élevée, tandis que les fibres les plus fines présentent des propriétés inverses.

Les phases compensatrices peuvent ainsi produire certains effets physiologiques du fait d'une capture de l'impulsion par certaines fibres nerveuses, potentiellement celles dont le seuil d'excitation est le plus bas. Il faut également tenir compte du fait que les fibres peu profondes, proches des électrodes, recevront plus de courant que les mêmes fibres situées en profondeur du nerf, et sont donc susceptibles d'être également activées même si leur seuil d'excitation est plus élevé.

Un premier but de l'invention est donc d'assurer une capture lors des phases de stimulation des fibres que l'on souhaite activer, tout en évitant une telle capture lors des phases compensatrices.

D'autres effets inattendus peuvent être observés sur des organes innervés par le nerf stimulé. Typiquement, on sait que le rythme cardiaque est ralenti par une stimulation du nerf vague, mais on constate qu'un ralentissement supplémentaire peut être produit par les fibres parasympathiques (fibres de type B) excitées par les phases compensatrices. Une stimulation multiphasique par des trains impulsionnels comportant des phases de stimulation et des phases compensatrices induit ainsi une réduction importante du rythme cardiaque, plus importante que celle que l'on aurait constatée avec une simple stimulation monophasique ne comportant pas de phases compensatrices.

Un deuxième but de l'invention est donc de pouvoir évaluer et contrôler la réponse physiologique produite le cas échéant par les phases compensatrices.

Enfin, à chaque fois qu'une neurostimulation est appliquée par le générateur du dispositif, celui-ci entre dans une période réfractaire absolue pendant laquelle est opérée une déconnexion ou *blanking* des circuits de détection, notamment de l'activité cardiaque et de l'électroneurogramme, et c'est seulement à l'issue de cette période de *blanking* que les circuits de détection et de mesure des potentiels physiologiques sont réactivés.

Un troisième but de l'invention est de permettre une réduction à un minimum de la durée des phases compensatrices (précharge, postcharge et décharge passive), de manière à réduire d'autant la durée de la période de *blanking* et permettre ainsi un retour rapide du dispositif à un fonctionnement normal, c'est-à-dire avec toutes les fonctions de détection des potentiels physiologiques. On notera toutefois que si l'on réduit la durée d'une impulsion de précharge, il faudra en augmenter d'autant l'amplitude pour conserver la même valeur de charge électrique, avec pour conséquence le risque d'induire des effets physiologiques qui ne seraient pas apparus avec une impulsion plus longue et de moindre amplitude.

L'idée de base de l'invention consiste, pour atteindre les buts précités, à prévoir un circuit de détection d'un paramètre physiologique et/ou physique et à produire un train impulsionnel de test afin de discriminer et évaluer l'apparition d'un éventuel effet physiologique produit par les phases compensatrices, puis piloter le générateur de neurostimulation en fonction du résultat du test de manière à adapter les paramètres de la (des) phase(s) compensatrice(s) active(s), c'est-à-dire de la (des) impulsion(s) de précharge ou postcharge, ou encore de décharge passive.

Le paramètre physiologique et/ou physique peut être un paramètre d'activité électrique du coeur, par exemple le rythme cardiaque HR (*Heart Rate*) calculé à partir des intervalles RR d'un signal d'électrogramme endocavitaire EGM. Il peut s'agir également d'un paramètre d'activité électrique du système nerveux dérivé d'un signal d'électroneurogramme ENG, d'un paramètre de fréquence respiratoire (ventilation-minute MV), de courant électrique traversant un tissu, d'accélération du corps du patient (accélération G), de pression sanguine, d'accélération endocardiaque EA, etc. Dans une autre mise en oeuvre, la (les) phase(s) compensatrice(s) active(s) peu(ven)t être ajustée(s) sur le niveau de la décharge passive, afin d'équilibrer les charges.

Selon le mode de réalisation mis en oeuvre et l'application particulière visée, le train impulsionnel multiphasique de test peut inclure, ou non, une ou plusieurs impulsions de stimulation. En d'autres termes, selon le cas, le test peut être effectué avec des impulsions de neurostimulation combinées à des impulsions de précharge, ou seulement avec des impulsions de précharge, sans neurostimulation.

Le principe de l'invention peut être appliqué soit à un système d'électrodes simples (notamment bipolaire, quasi-tripolaire ou tripolaire), mais aussi à un système combinant plusieurs électrodes de stimulation (notamment multipolaire). L'invention peut enfin s'appliquer à tout système de stimulation biologique délivrée en courant (notamment stimulation myo-cardique, musculaire).

Plus précisément, l'invention a pour objet un dispositif médical actif comprenant, de manière en elle-même connue et divulguée notamment par le US2010/0114198 A1 précité :
- un générateur de neurostimulation électrique fonctionnelle, comprenant des moyens pour produire en sortie des trains impulsionnels multiphasiques de neurostimulation ;
- une sonde de stimulation, destinée à être disposée sur ou à proximité d'une structure du système nerveux d'un patient porteur du dispositif pour l'application à cette structure des trains impulsionnels de neurostimulation ;
- au moins un capteur délivrant un signal de contrôle représentatif d'un paramètre physiologique et/ou physique susceptible d'être influencé par la délivrance des trains impulsionnels de neurostimulation ; et
- un circuit de contrôle automatique de compensation de charge, comprenant des moyens pour recevoir en entrée le signal de contrôle délivré par le au moins un capteur.

De façon caractéristique de l'invention, chaque train impulsionnel comprend au moins une impulsion de stimulation et au moins une impulsion compensatrice, cette dernière comprenant : une impulsion de précharge précédant l'impulsion de stimulation ; et/ou une impulsion de postcharge suivant l'impulsion de stimulation ; une impulsion de décharge passive terminant le train impulsionnel ; ou une combinaison des précédentes. Les impulsions de stimulation et compensatrice sont des impulsions de courant dont le sens, la durée et l'amplitude sont contrôlés, et les impulsions compensatrices procurent une charge électrique cumulée compensant la charge électrique de la au moins une impulsion de stimulation.

De plus, le circuit de contrôle automatique de compensation de charge comprend en outre des moyens pour déterminer une amplitude et/ou une durée d'impulsion compensatrice en fonction d'au moins un critère prédéterminé, et des moyens pour délivrer au générateur un signal de pilotage des impulsions compensatrices à produire en sortie par celui-ci.

Selon diverses caractéristiques subsidiaires avantageuses :
- le circuit de contrôle automatique de compensation de charge est un circuit apte à : contrôler le générateur de manière à produire au moins un train impulsionnel de test ; recueillir le signal de contrôle après la fin du au moins un train impulsionnel de test ; déterminer d'après le niveau du signal de contrôle si un effet physiologique et/ou physique est ou non produit par l'application du train impulsionnel de test ; et déterminer l'amplitude et/ou la durée des impulsions compensatrices des trains impulsionnels suivants en fonction du résultat de la détermination ;
- le train impulsionnel de test comprend une impulsion de précharge suivie d'au moins une impulsion de stimulation ; dans ce cas, le circuit de contrôle automatique de compensation de charge est avantageusement apte à délivrer un unique train impulsionnel de test et à recueillir le signal de contrôle immédiatement après la fin du train impulsionnel de test ;
- en variante, le train impulsionnel de test comprend une impulsion de précharge mais ne comprend pas d'impulsion de stimulation ; dans ce cas, le circuit de contrôle automatique de compensation de charge est avantageusement apte à délivrer une pluralité de trains impulsionnels de test successifs et à recueillir le signal de contrôle après la fin du dernier train impulsionnel de test de ladite pluralité ;
- le circuit de contrôle automatique de compensation de charge est apte à déterminer l'amplitude et/ou la durée des impulsions compensatrices des trains impulsionnels courants en fonction de l'amplitude maximale de l'impulsion de décharge passive mesurée pour le train impulsionnel de test ;
- le circuit de contrôle automatique de compensation de charge est en outre apte à commander un intervalle temporel entre la fin de l'impulsion de stimulation et la fermeture d'interrupteurs de mise au même potentiel des électrodes de stimulation, de manière à moduler l'instant de début de l'impulsion de décharge passive ;
- le circuit de contrôle automatique de compensation de charge est en outre apte à commander un intervalle temporel entre la fin de l'impulsion de précharge et le début de l'impulsion de stimulation ;
- le circuit de contrôle automatique de compensation de charge est en outre apte à commander un intervalle temporel entre la fermeture et l'ouverture d'interrupteurs de mise au même potentiel des électrodes de stimulation, de manière à moduler la durée de l'impulsion de décharge passive ;
- le capteur est un capteur de mesure d'un paramètre du groupe formé par : activité électrique du coeur, activité électrique du système nerveux, rythme cardiaque, fréquence respiratoire, courant électrique traversant un tissu, accélération du corps du patient, pression sanguine, accélération endocardiaque, position du patient.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 est une vue générale d'un patient implanté avec un dispositif VNS (stimulation du nerf vague), ce dispositif comprenant en outre des moyens de recueil du rythme cardiaque au moyen d'une sonde endocavitaire.
La Figure 2 montre de façon schématique les principaux blocs fonctionnels du générateur du dispositif VNS de la Figure 1 pour la mise en oeuvre de l'invention.
La Figure 3 illustre une séquence de délivrance de trains impulsionnels de thérapie VNS, synchronisés sur la détection des battements cardiaques.
La Figure 4 illustre de façon plus précise un exemple de train impulsionnel de neurostimulation, avec les effets recherchés sur l'électroneurogramme ENG mais aussi les effets indésirables produits par chacune des impulsions de ce train impulsionnel.
La Figure 5 illustre un exemple d'effet produit sur le rythme cardiaque par des trains impulsionnels de neurostimulation, respectivement avec et sans impulsion de précharge.
La Figure 6 est une représentation d'une caractéristique "isocharge", pour trois valeurs de charge produites par une impulsion de courant en fonction de la durée PW de cette impulsion.
La Figure 7 est un diagramme de Lapicque décrivant la relation entre les caractéristiques d'une impulsion de stimulation et une réponse physiologique résultante.
Les Figures 8a et 8b sont des représentations schématiques des circuits équivalents du générateur d'impulsions (impulsions de stimulation ou bien de précharge) et de l'interface avec les tissus recevant ces impulsions, respectivement dans une phase d'application d'une impulsion et dans une phase de décharge passive.
La Figure 9 est une représentation en superposition, caractéristique de l'invention, de l'allure d'une décharge passive avec un diagramme de Lapicque tel que celui de la Figure 7.
Les Figures 10a, 10b et 10c montrent trois exemples de train impulsionnel multiphasique avec, pour une même impulsion de stimulation, des impulsions de précharge d'amplitude ou de durée différentes, engendrant des impulsions de décharge passive de niveau différent.
La Figure 11 illustre, dans le cadre d'une application de la technique de l'invention au contrôle du rythme cardiaque, une modélisation de la loi de Lapicque pour une cible donnée de réduction de la fréquence cardiaque.
La Figure 12 illustre un neurogramme montrant les variations du potentiel d'action composite pour différentes valeurs du courant de stimulation.
La Figure 13 illustre des caractéristiques selon la loi de Lapicque, correspondant à des cibles différentes de réduction de la fréquence cardiaque.
La Figure 14 représente des caractéristiques isocharge déterminées pour différentes valeurs de charge produites par l'impulsion de décharge passive d'une phase compensatrice, en superposition avec une caractéristique selon la loi de Lapicque.
La Figure 15 est un organigramme schématique des principales étapes de mise en oeuvre de la technique de l'invention.
La Figure 16 illustre l'application de deux trains impulsionnels de neurostimulation délivrés en synchronisme avec le rythme cardiaque.
La Figure 17 illustre l'application de trois modalités de VNS, d'un train impulsionnel multiphasique de neurostimulation ou d'une séquence de test où les trains impulsionnels appliqués présentent différents motifs de phases compensatrices.
La Figure 18 illustre une séquence de test complète, délivrée en synchronisme avec le rythme cardiaque.
La Figure 19 illustre, sur une superposition de la caractéristique isocharge et du diagramme de Lapicque, une technique de recherche du point optimal par un algorithme de type itératif.
La Figure 20 est homologue de la Figure 19, pour un algorithme de type pseudo-stochastique.
La Figure 21 est un organigramme illustrant les principales étapes d'une technique de recherche du point optimal par un algorithme de type pas à pas.
La Figure 22 illustre les variations du rythme cardiaque induites par des salves successives d'une séquence de test.
La Figure 23 est un organigramme illustrant les principales étapes de recherche du point optimal par test de grandes, puis de petites, variations sur la courbe courant/largeur d'impulsion.
Les Figures 24 et 25 illustrent, sur un diagramme courant/largeur d'impulsion, la technique de recherche de la Figure 23 sur une caractéristique isocharge, respectivement pour des grandes variations et pour des petites variations sur cette courbe.
La Figure 26 est un organigramme général présentant les étapes successives de recherche de la configuration optimale selon l'invention.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type neurostimulateur, myostimulateur, stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Equilia, Reply* et *Paradym* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé de l'invention est mis en oeuvre par des moyens matériels et logiciels, au moyen d'algorithmes appropriés exécutés par un microcontrôleur ou un processeur numérique de signal et d'un circuit de stimulation en courant. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel.

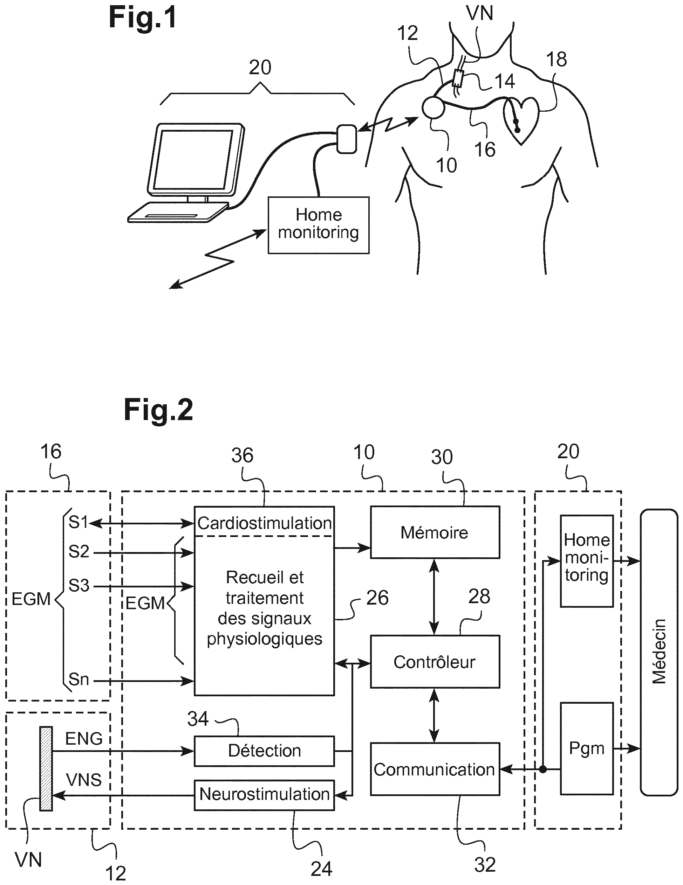

Sur la Figure 1, la référence 10 désigne le boitier d'un générateur implantable de stimulation du nerf vague (VNS). Cette stimulation est délivrée par une sonde 12 portant à sa partie distale un manchon 14 implanté autour du nerf vague VN, ce manchon 14 étant pourvu d'électrodes susceptibles de stimuler le nerf vague par application de salves d'impulsions VNS produites par le générateur 10. Le générateur 10 comprend également des moyens de recueil du rythme cardiaque, qui sont dans cet exemple constitués d'une sonde cardiaque endocavitaire 16 pourvue à son extrémité distale d'électrodes 18 de détection de l'activité électrique du ventricule. Cette sonde 16 recueille ainsi des signaux d'électrogramme endocavitaire EGM permettant, à chaque cycle cardiaque, de détecter une onde R de dépolarisation ventriculaire.

Dans d'autres mises en oeuvre, le signal détecté est un signal physiologique, notamment la ventilation-minute ou l'électroneurogramme. Dans une autre mise en oeuvre, la (les) phase(s) compensatrice(s) active(s) peut(vent) être ajustée(s) sur le niveau de la décharge passive, afin d'équilibrer les charges

De préférence, la stimulation VNS est appliquée au nerf vague droit au niveau cervical. Dans d'autres mises en oeuvre, la stimulation VNS est appliquée à gauche et/ou sur une branche du nerf vague. Dans d'autres mises en oeuvre encore, la neurostimulation est appliquée à proximité d'un nerf ou une de ses branches du système parasympathique.

Le générateur 10 peut être éventuellement conçu pour délivrer, outre les impulsions de neurostimulation appliquées au nerf vague VN, des impulsions de cardiostimulation appliquées aux électrodes 18 (et éventuellement à d'autres électrodes, ventriculaires ou auriculaires) pour permettre le traitement de troubles bradycardiques, tachycardiques ou de de la synchronisation ventriculaire. Cette fonction n'est toutefois pas nécessaire pour la mise en oeuvre de l'invention, qui concerne seulement la thérapie de neurostimulation et l'analyse des effets de cette thérapie notamment (mais non nécessairement) à partir des signaux cardiaques recueillis par des électrodes endocardiaques, celles-ci pouvant être utilisées indifféremment en simples électrodes de détection de signal, ou bien en électrodes de détection/stimulation.

L'ensemble peut être en outre muni d'un système de communication 20 comprenant un boitier externe de télémétrie permettant une communication avec le générateur 10 pour assurer le transfert et la réception de données avec ce dernier. Le boitier de communication peut être par ailleurs connecté à un dispositif externe tel qu'un programmateur ou bien un dispositif de *home monitoring* pour le suivi à distance du patient par télétransmission à un site distant des données recueillies et enregistrées par le générateur.

On notera par ailleurs que, dans l'exemple illustré, la technique de l'invention se fonde principalement ou exclusivement sur l'analyse de signaux cardiaques (signaux EGM en l'espèce) pour l'analyse de l'effet physiologique de la neurostimulation et l'ajustement des paramètres de fonctionnement du générateur 10. Cette analyse peut toutefois, en variante ou en complément, se fonder sur des signaux produits par des capteurs relatifs à d'autres paramètres physiques ou physiologiques tels que : activité électrique du système nerveux (par analyse d'un électroneurogramme ENG), fréquence respiratoire (notamment le signal de ventilation-minute MV), courant électrique traversant un tissu, accélération du corps du patient (information donnée par un accéléromètre intégré au générateur implanté), pression sanguine, accélération endocardiaque EA (par un capteur d'accélération intégré à la sonde cardiaque 16), etc. De façon générale, il s'agit de disposer d'un signal représentatif d'au moins un paramètre physiologique ou physique susceptible d'être influencé par la délivrance des impulsions de stimulation, en l'espèce par la délivrance des impulsions VNS.

La Figure 2 illustre de façon générale les différents blocs fonctionnels du générateur 10 de la Figure 1 permettant la mise en oeuvre de l'invention, avec les signaux d'entrée et de sortie.

Le générateur 10 comprend un circuit 24 de production d'impulsions VNS, qui sont des impulsions de courant dont i) le sens, ii) l'amplitude du courant et iii) la durée (largeur d'impulsion PW) sont contrôlés en fonction de paramètres prédéterminés. Ces impulsions sont appliquées au nerf vague VN par la sonde 12, de la manière exposée plus haut.

Dans une autre application, la configuration des pôles de stimulation peut être programmable, notamment dans un dispositif multipolaire disposant de plusieurs contacts électriques.

Le générateur 10 comprend également un circuit 26 de recueil et de traitement de signaux physiologiques et/ou physiques, qui dans cet exemple sont des signaux d'activité électrique du coeur délivrés par une ou plusieurs électrodes de la sonde endocardiaque 16 (signaux d'électrogramme endocavitaire EGM) recueillis sur une ou plusieurs voies S1 ... Sn. Comme indiqué plus haut, ce circuit peut également recueillir d'autres signaux tels que : électroneurogramme ENG, accélération cardiaque EA, activité respiratoire (signal MV), accélération du corps du patient (signal G), niveau pic de la décharge passive, etc.

Les circuits 24 et 26 sont interfacés à un contrôleur 28 et à une mémoire 30, pour permettre notamment le pilotage du circuit de neurostimulation 24, de la manière qui sera décrite plus bas, en fonction des signaux physiologiques et/ou physiques recueillis par le circuit 26.

Le générateur peut également comprendre, comme indiqué plus haut, une interface de communication 32 pour l'échange de données avec des dispositifs extérieurs 20 tels qu'un programmateur pour permettre l'interaction avec un médecin à proximité du patient, et/ou un dispositif de *home monitoring* pour la télétransmission de données à un site distant où ces données seront analysées.

Le générateur 10 peut également comprendre un circuit 34 de détection de signaux d'activité nerveuse (électroneurogramme ENG), en particulier des signaux recueillis par les électrodes du manchon 14 en contact avec le nerf vague VN.

Enfin, le générateur 10 peut être le cas échéant pourvu d'un circuit de cardiostimulation 36 apte à délivrer au coeur du patient, via la sonde endocavitaire 16, des impulsions de thérapie antibradycardique, antitachy-cardique et/ou de resynchronisation ventriculaire.

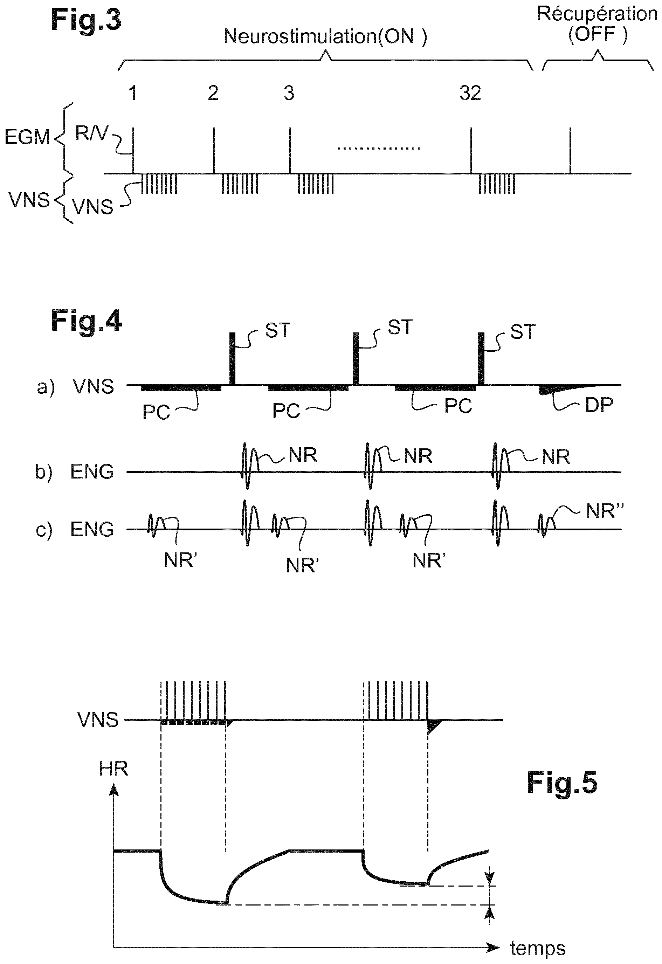

On va maintenant expliquer, en référence aux Figures 3 et 4, le type d'impulsions produites par le circuit de neurostimulation 24.

La Figure 3 illustre une séquence de délivrance de trains impulsionnels de thérapie VNS, synchronisés sur la détection des battements cardiaques, plus précisément sur la détection, par le circuit 26, d'une dépolarisation ventriculaire stimulée (évènement V) ou spontanée (évènement R).

Typiquement, ces salves d'impulsion VNS sont délivrées pendant des périodes "ON" de durée prédéterminée, entrecoupées de périodes "OFF" pendant laquelle aucune stimulation VNS n'est délivrée. Ces salves de stimulation VNS, pilotées par le contrôleur 28, peuvent être définies par de nombreux paramètres de configuration tels que :
- synchronisme : la stimulation VNS peut être synchrone (comme dans le présent exemple) ou non des événements cardiaques ;
- dans le cas d'une stimulation synchrone, ratio entre les stimulations VNS et les évènements cardiaques, un ratio de 1:1 indiquant une stimulation VNS à chaque évènement cardiaque détecté, un ratio de 1:4 indiquant une impulsion de stimulation VNS tous les quatre évènements cardiaques, etc. ;
- toujours dans le cas d'une stimulation VNS synchrone, durée de l'intervalle R-VNS, paramètre décrivant le retard entre la détection de l'évènement cardiaque et le début de la salve d'impulsions VNS, nombre d'impulsions VNS dans la salve ;
- courant délivré au nerf vague (courant pic ou courant moyen) ;
- fréquence des impulsions ;
- largeur des impulsions ;
- rapport cyclique (*duty cycle*) de l'alternance des périodes "ON" de stimulation et "OFF" de non-stimulation.

Comme indiqué en introduction, l'invention concerne spécifiquement le cas où la stimulation est appliquée sous forme d'une succession de trains impulsionnels multiphasiques comprenant chacun :
- au moins une impulsion de précharge (et/ou de postcharge, ce cas étant assimilé à une impulsion de précharge), délivrée de façon contrôlée par le générateur 24 ;
- au moins une impulsion de neurostimulation proprement dite, également délivrée par le circuit de neurostimulation 24 ; et
- une impulsion apparaissant sous forme d'une décharge passive, spontanée, dans la bioimpédance formée par les tissus au niveau du site de stimulation.

L'impulsion de stimulation est délivrée sous forme d'un courant de sens prédéterminé et constitue une "phase de stimulation", tandis que les impulsions de précharge se présentent sous forme d'un courant de sens opposé, correspondant à une "phase compensatrice". La décharge passive est appliquée après les impulsions de précharge, de stimulation et de postcharge. Théoriquement, la charge électrique cumulée de la (des) phase(s) compensatrice(s) est égale, au signe près, à la charge électrique de l'impulsion de stimulation.

La Figure 4 illustre en (a) un exemple de train impulsionnel multiphasique de neurostimulation, avec trois impulsions de stimulation ST précédées chacune d'une impulsion de précharge PC, le train impulsionnel s'achevant par une impulsion de décharge passive DP.

Comme exposé en introduction, l'impulsion de précharge PC (qui peut être aussi une impulsion de postcharge produite après l'impulsion de stimulation ST) est une impulsion de sens (sens du courant délivré) contraire de celui de l'impulsion de neurostimulation ST, et délivre aux tissus une quantité de charge approximativement égale à celle de l'impulsion de stimulation ST, de façon à assurer la neutralité électrique finale et éviter une accumulation excessive de charges à l'interface de stimulation du nerf vague. À la fin du train impulsionnel, la décharge passive DP correspond à l'élimination des charges résiduelles encore éventuellement présentes au site de stimulation.

La polarité de la décharge passive DP est *a priori* inconnue, car elle dépend des charges résiduelles à l'interface électrodes-tissus. Il faut considérer deux phénomènes : 1°) l'imprécision inhérente à tout système ne permet pas un équilibre parfait des charges délivrées, en valeur absolue, de l'impulsion de stimulation et des charges des précharges et post-charges, et 2°) certaines charges ont pu se dissiper dans le milieu ambiant.

En (b) et (c) des relevés d'électroneurogramme ENG montrent :
- les effets recherchés NR sur le système nerveux, ces effets résultant de l'impulsion de stimulation proprement dite ST, visibles en b),
- mais également, comme on peut le voir en (c), les effets indésirables NR' et/ou NR" produits respectivement par les impulsions de précharge PC et par la décharge passive DP.

Le but de l'invention est de proposer une technique permettant de contrôler ces réponses non attendues NR' et NR", généralement pour tenter de les faire disparaitre par un paramétrage approprié des impulsions de précharge PC.

Dans une variante, au lieu de chercher à réduire ou éliminer ces réponses non attendues, on cherchera à contrôler ces réponses sur le système nerveux produites par la phase compensatrice de la neurostimulation, de manière à maitriser la réponse globale produite à la fois par les impulsions de précharge PC et les impulsions de stimulation ST.

La Figure 5 illustre un exemple d'effet produit sur le rythme cardiaque par des trains impulsionnels de neurostimulation, avec un premier train contenant des impulsions compensatrices de précharge et le deuxième train dans lequel ces impulsions compensatrices de précharge n'ont pas été appliquées.

Le second chronogramme montre que les impulsions de précharge produisent une réduction additionnelle du rythme cardiaque HR (c'est-à-dire un allongement du délai RR entre deux cycles cardiaques successifs, par rapport à un train impulsionnel sans impulsion de précharge).

Ce phénomène est dû à la capture de fibres parasympathiques, notamment les fibres de type A et/ou B du nerf vague, agissant sur la fréquence cardiaque par les phases compensatrices. Dans ce cas, l'utilisation d'impulsions compensatrices de précharge a pour effet une amplification de l'effet physiologique produit par la neurostimulation, phénomène dont il faudra tenir compte pour l'ajustement des paramètres de la neurostimulation.

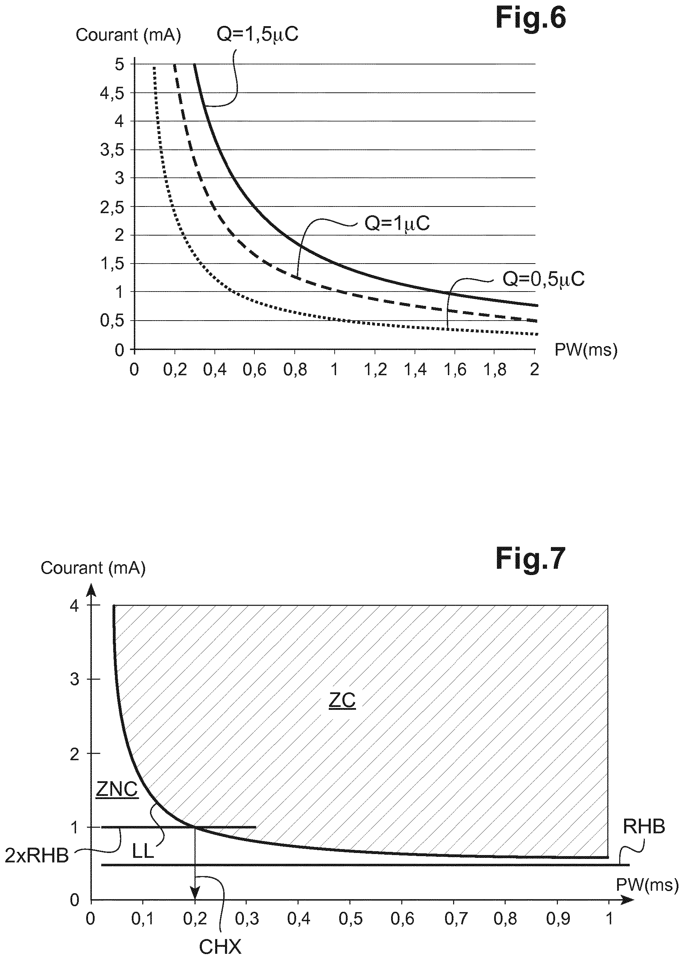

La Figure 6 est une représentation d'une caractéristique "isocharge" illustrant la relation entre la durée PW de l'impulsion et l'intensité du courant, à charge constante, pour trois valeurs de charge différentes Q=0,5 µC, Q=1 µC et Q=1,5 µC produites par une impulsion de courant. La charge étant le produit de la durée de l'impulsion (que l'on suppose rectangulaire) par l'intensité du courant, ces courbes se présentent sous forme d'hyperboles.

Ainsi, une impulsion de stimulation de 1 ms/1 mA, soit 1 µC, pourra être compensée par une impulsion de précharge de 2 ms/0,5 mA, ou de 3 ms/0,33 mA, ou de 4 ms/0,25 mA, ou de 5 ms/0,20 mA, ou de 6 ms/0,17 mA, etc. On cherche bien entendu, pour minimiser les effets ou annuler les effets physiologiques produits par l'impulsion de précharge, à allonger celle-ci à une valeur de durée d'impulsion permettant de réduire d'autant l'amplitude du courant compensateur, et donc les effets physiologiques imprévus attachés à cette impulsion.

La Figure 7 est une représentation, dans un espace courant/durée d'impulsion, du diagramme dit "loi de Lapicque" décrivant la relation entre les caractéristiques de courant et de durée d'impulsion d'une impulsion, et la réponse physiologique induite (ou non) par cette impulsion.

Lorsque la réponse physiologique est un paramètre cardiaque tel que la fréquence cardiaque HR, la réponse physiologique est du type "tout ou rien", c'est-à-dire que, selon le cas, l'impulsion produit soit une dépolarisation complète du coeur (zone de capture ZC), soit une absence complète de dépolarisation (zone de non-capture ZNC). La frontière entre les deux zones ZC et ZNC, c'est-à-dire la limite inférieure de la zone de capture ZC, est la caractéristique LL dite "loi de Lapicque".

Cette courbe de Lapicque est typiquement caractérisée par deux descripteurs, à savoir :
- en ordonnée, le courant dit "rhéobase" RHB, qui est le niveau de courant auquel aucune capture ne sera jamais possible quelle que soit la largeur des impulsions (asymptote horizontale de la courbe LL) ; et
- en abscisse, une durée dite "chronaxie", CHX qui est la durée d'impulsion correspondant, par convention, au double de la rhéobase RHB.

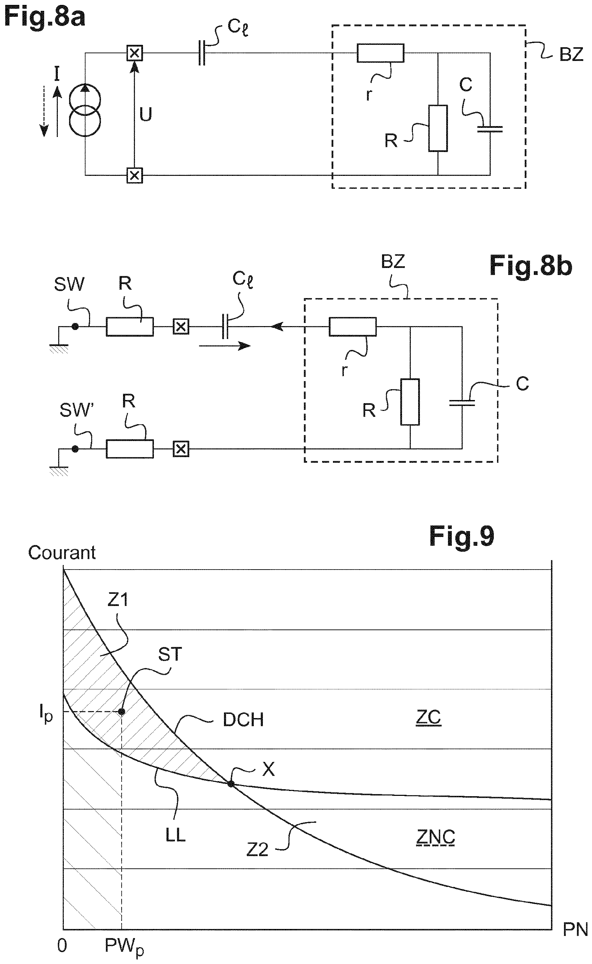

Les Figures 8a et 8b sont des représentations schématiques des circuits équivalents du générateur d'impulsions (impulsions de stimulation ST ou bien impulsions de précharge PC) et de l'interface avec les tissus recevant ces impulsions, respectivement dans une phase d'application d'une impulsion et dans une phase de décharge passive.

Dans une phase d'application d'une impulsion (Figure 8a), le générateur produit un courant I dans un sens ou dans l'autre (selon qu'il s'agit d'une impulsion de précharge ou d'une impulsion de stimulation), courant qui est appliqué à l'interface, représentée de façon typique par une bioimpédance BZ comprenant une capacité C avec une résistance parallèle R et une résistance série r. Cette bioimpédance BZ est couplée au générateur par une capacité de liaison Cℓ.

Lors de la décharge passive (Figure 8b), qui fait suite à l'application de l'impulsion ou des impulsions de neurostimulation, les électrodes de stimulation sont mises au même potentiel électrique (interrupteurs SW et SW' de résistance équivalente R et R', typiquement mais non exclusivement à la masse) et la charge accumulée dans la capacité de liaison Cℓ produit une différence de potentiel qui provoque la circulation d'un courant dans la boucle constituée par la bioimpédance BZ, la capacité de liaison Cℓ et les résistances internes R et R' des interrupteurs SW et SW'. Ce courant décroit progressivement, selon une loi de décharge d'une capacité dans une impédance complexe comprenant des composantes à la fois capacitives et résistives.

### Principes de base de l'invention

Le point de départ de l'invention réside dans une superposition d'une courbe isocharge (comme en Figure 6) et d'une courbe de type loi de Lapicque (comme en Figure 7), courbes qui ont les mêmes grandeurs en abscisse et en ordonnée, à savoir la largeur d'impulsion PW et l'intensité du courant.

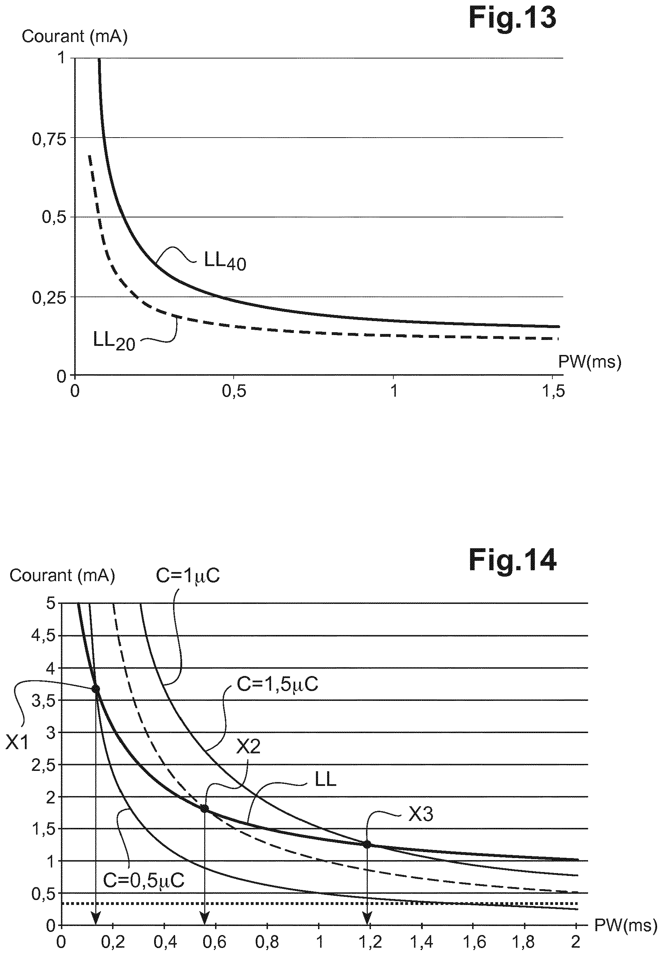

La Figure 14, détaillée plus loin, illustre une telle représentation avec superposition de la loi de Lapicque LL et de plusieurs caractéristiques isocharge.

Un second aspect de l'invention consiste à prendre en compte les effets d'une décharge passive sur les tissus en superposant une courbe de Lapicque et une courbe de décharge passive, courbes qui ont les mêmes grandeurs en abscisse et en ordonnée, à savoir la largeur d'impulsion PW et l'intensité du courant.

La Figure 9 représente les paramètres d'une impulsion de durée PWₚ et d'intensité Iₚ correspondant à un équivalent d'une décharge passive DCH. Dans les faits, la décharge passive est en exponentielle négative, correspondant à la décharge de l'interface tissus-électrodes ainsi que des capacités de liaison (en première approximation équivalente à la décharge d'une capacité sur une résistance).

Dans l'exemple illustré, la courbe DCH correspondant à une décharge passive se trouve toujours au-dessus de l'aire correspondant à l'impulsion {PWₚ, Iₚ}, ce qui signifie que les effets biologiques de la décharge passive seront toujours au moins aussi importants que les effets biologiques de l'impulsion. De plus, le point ST correspondant à l'impulsion {PWₚ, Iₚ} se situe nettement au-dessus de la courbe correspondant à la loi de Lapicque LL, ce qui signifie que cette impulsion produira effectivement une stimulation (le point ST étant situé dans la zone de capture ZC). On peut donc conclure, dans cet exemple, que la décharge passive, par rapport à la courbe de Lapicque LL, produira également un effet physiologique de stimulation.

La zone hachurée Z1 indique une zone qui devra être nécessairement évitée par la décharge passive, car cette dernière sera susceptible d'exciter des fibres nerveuses et donc de produire un effet physiologique indésirable.

Le but de l'invention est de proposer une technique permettant d'ajuster les caractéristiques de l'impulsion de précharge (ou postcharge) de manière à contrôler le niveau maximal de l'impulsion de décharge passive, typiquement pour minimiser celle-ci en deçà du seuil de stimulation, voire même la faire pratiquement disparaitre, par un équilibrage optimal de la quantité de charge de l'impulsion de précharge par rapport à l'impulsion de stimulation.

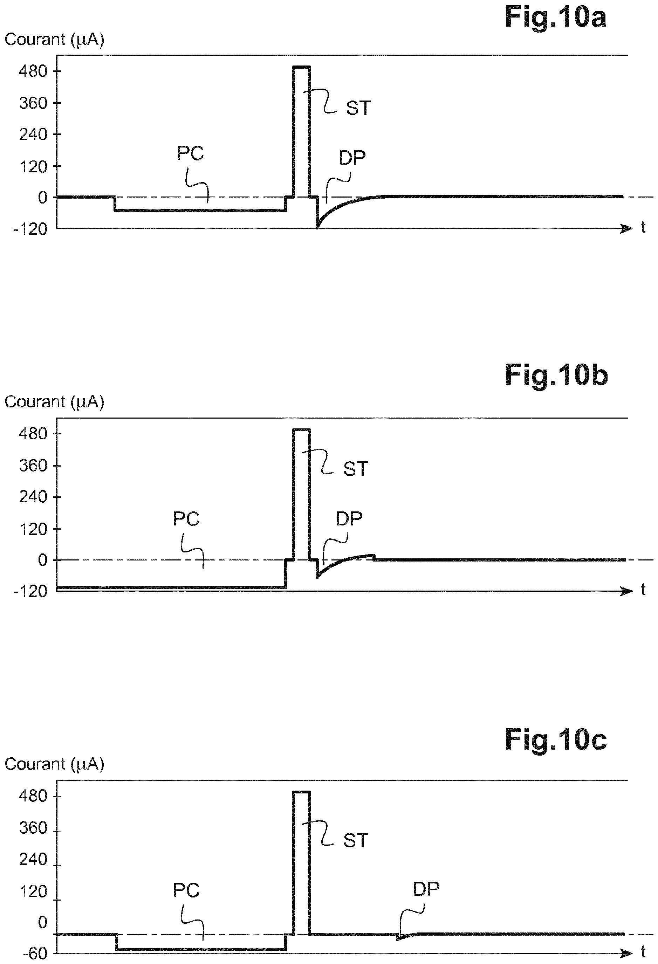

Les Figures 10a, 10b et 10c montrent trois exemples de train impulsionnel multiphasique comprenant chacun une même impulsion de stimulation ST, mais avec des impulsions de précharge respectives PC d'amplitude ou de durée différentes, engendrant des impulsions de décharge passive DP de niveaux et de configurations différentes.

Sur les trois figures, l'impulsion de stimulation ST est une impulsion de 0,5 mA/300 µs.

Sur la Figure 10a, on a généré une impulsion de précharge PC de -50 µA/3 ms correspondant à une valeur de charge égale, au signe près, à celle de l'impulsion de stimulation, donc une précharge a priori équilibrée.

On constate dans ce cas l'apparition d'une décharge passive DP pour laquelle le front initial produit un pic de courant d'environ -110 µA, soit plus du double de la valeur du courant de précharge (-50 µA). Cette impulsion de décharge passive va donc très vraisemblablement produire un effet physiologique indésirable.

Or cette décharge est une décharge capacitive passive, non contrôlée en soi, qui ne dépend en fait que du niveau des charges appliquées à l'interface avec le nerf, de la bioimpédance de celui-ci, etc.

L'idée de base de l'invention consiste à moduler l'amplitude et/ou la durée de l'impulsion de précharge PC, donc la valeur de la charge appliquée préalablement à la stimulation, de manière à agir indirectement sur le niveau du pic de courant de la décharge passive DP, dès lors que l'on constate que la précharge appliquée a un effet sur le niveau de l'impulsion de décharge passive DP.

Ainsi, sur la Figure 10b, on a généré une impulsion de précharge PC de -100 µA/4 ms, qui n'est donc a *priori* pas équilibrée par rapport à l'impulsion de stimulation ST, qui est toujours de 0,5 mA/300 µs. On constate toutefois dans ce cas une diminution importante du pic de la décharge passive DP, avec un front initial de l'ordre de -65 µA seulement, inférieur au niveau de l'impulsion de précharge (-100 µA) et deux fois moindre que ce que l'on avait constaté (-110 µA) avec une impulsion de précharge équilibrée (cas de la Figure 10a).

Ainsi, en modulant l'impulsion de précharge PC, on peut arriver à maitriser la décharge passive DP pour amener celle-ci à un niveau auquel elle ne produira pas d'effets physiologiques indésirables, ou des effets négligeables.

D'autres séquencements de l'impulsion de précharge PC peuvent être envisagés pour réduire l'amplitude de la décharge passive DP, en modulant par exemple :
- le séquencement entre la fin de l'impulsion de précharge PC et le début de l'impulsion de stimulation ST ;
- le séquencement entre la fin de l'impulsion de stimulation ST et le début de l'impulsion de décharge passive DP (cet instant est en effet contrôlable, car il correspond à la fermeture des interrupteurs de mise au même potentiel électrique des électrodes de stimulation) ;
- le séquencement entre le début et la fin de l'impulsion de décharge passive DP (intervalle entre la fermeture et l'ouverture des interrupteurs de mise au même potentiel électrique).

Comme illustré dans l'exemple de la figure 10c, il est même possible de réduire pratiquement à néant l'impulsion de décharge : dans cet exemple, on a utilisé la même impulsion de précharge PC, équilibrée, que celle de la Figure 10a (à savoir une impulsion de précharge de -50 µA/3 µs), mais on a prolongé le délai entre la fin de l'impulsion de stimulation ST et le début de la décharge passive DP, à une valeur de l'ordre de 1,6 ms au lieu de 0,1 ms comme sur les Figures 10a et 10b. On constate que ce paramétrage permet de faire pratiquement disparaitre l'impulsion de décharge passive DP.

### Mise en oeuvre clinique de l'invention

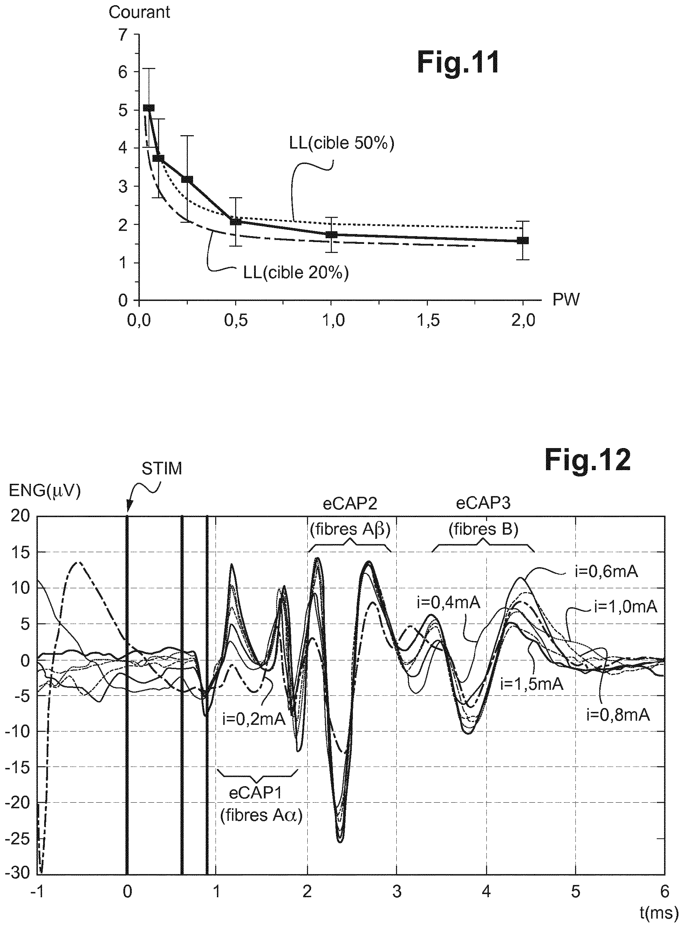

On va maintenant décrire, en référence aux Figures 11 à 26, un certain nombre d'exemples de mise en oeuvre cliniques de l'invention.

Une *première application clinique* est le contrôle du rythme cardiaque.

La Figure 11 illustre, dans le cadre d'une telle application, une modélisation de la loi de Lapicque pour une cible donnée de réduction de la fréquence cardiaque. Comme on l'a indiqué en introduction, le rythme cardiaque HR est sous le contrôle du système nerveux autonome, le système sympathique agissant par augmentation du rythme cardiaque et le système parasympathique (auquel appartient le nerf vague) agissant par ralentissement du rythme cardiaque.

En augmentant la charge de l'impulsion de neurostimulation délivrée au nerf vague (courant et largeur d'impulsion), il est possible d'agir sur les variations de la fréquence cardiaque HR par un choix approprié de paramètres de VNS (notamment courant et largeur d'impulsion). Toutefois, à la différence de la stimulation cardiaque directe, c'est-à-dire d'une application d'impulsions de dépolarisation à des électrodes endocavitaires, les effets sur la diminution de la fréquence cardiaque HR dans le cas d'une neurostimulation sont des effets progressifs, de sorte que dans ce cas la loi de Lapicque ne suit pas un modèle de type "tout ou rien" tel qu'il avait été décrit plus haut, notamment en référence à la Figure 7.

La réponse physiologique attendue doit donc être quantifiée, par exemple une diminution de 20% de la fréquence cardiaque, cette réponse attendue constituant une cible de fréquence cardiaque qui est modélisable, comme illustré en Figure 11, sous forme d'une caractéristique {largeur d'impulsion, courant} de type loi de Lapicque LL. Pour un domaine situé au-dessus de cette courbe, la diminution de fréquence cardiaque sera supérieure à 20%, tandis que pour un point situé au-dessous de cette courbe, cette diminution sera inférieure à 20 %. Cette limite définit un critère physiologique cible.

Pour une cible moindre, par exemple 10 % de diminution de la fréquence cardiaque, la courbe de Lapicque LL' sera située dans une région plus basse de l'espace {largeur d'impulsion, courant}.

Une *deuxième application clinique,* illustrée en référence aux Figures 12 et 13, est celle de l'activation contrôlée d'une fibre nerveuse.

Comme on l'a indiqué en introduction, un nerf est constitué de plusieurs milliers d'axones, le nerf vague comprenant ainsi plus de 100 000 fibres. Ces fibres présentent des diamètres variables (allant de 25 µm à 0,2 µm pour les plus petites), et peuvent être ou non myélinisées. Elles conduisent toutes des potentiels d'actions, mais la vitesse de conduction dépend de leur diamètre (les plus grosses fibres conduisent plus vite les potentiels d'action) et du fait qu'elles sont ou non myélinisées (les fibres myélinisées conduisent plus vite les potentiels d'action). De plus, leur seuil de stimulation dépend également de ces mêmes paramètres (les plus grosses fibres et les fibres myélinisées ont des seuils d'activation plus bas, c'est-à-dire sont plus facilement excitables).

Si l'on délivre au nerf un stimulus électrique d'un niveau suffisant, celui-ci produira un potentiel d'action évoqué composite ou eCAP (*evoked Compound Action Potential*), qui est la somme spatiale de tous les potentiels d'action produits par les fibres excitées par le stimulus. Compte tenu de la diversité des fibres, les fibres les plus rapides (les plus grosses) produiront des potentiels d'action dès le début du eCAP, tandis que les fibres les plus lentes (celles de petit diamètre) produiront des potentiels d'action en fin de eCAP. De plus, dans la mesure où le seuil d'activation d'une fibre, en termes de charge électrique, dépend de son diamètre (plus grand est le diamètre plus faible est le seuil d'activation, et *vice versa*), plus le niveau du stimulus augmente plus les fibres activées seront nombreuses, de sorte que le eCAP augmentera, en amplitude pour un même type de fibres et en durée pour des fibres de caractéristiques différentes.

La Figure 12 montre ainsi un exemple d'électroneurogramme ENG illustrant les modifications du potentiel d'action évoqué composite pour différents niveaux de stimulus correspondant à des courants compris entre 0,2 et 1,5 mA. Ainsi, sur la Figure 12, plusieurs composantes du eCAP sont observables, les fibres Aα, Aβ et B (de la plus rapide à la plus lente) apparaissant plus ou moins proches de la stimulation du nerf.

Comme dans le cas du contrôle du rythme cardiaque, l'activation d'un nerf (eCAP) peut être représentée par une courbe de Lapicque, pour une valeur cible quantifiée. Dans ce cas, la valeur cible est exprimée en pourcentage d'une composante du eCAP par rapport à sa valeur maximale. Dans une mise en oeuvre, la valeur maximale sera celle mesurée par un système de recueil de l'ENG. Dans une autre mise en oeuvre, il s'agira d'une valeur typique prédéfinie. Par exemple, pour les fibres B, si le maximum mesuré est 20µV crête à crête, une cible de 20% correspondra à un eCAP de fibres B égal à 4µV. De même pour une valeur typique de 15µV, une cible de 20% correspondra à une cible de 3µV.

Ainsi, la Figure 13 illustre deux exemples de caractéristiques de Lapicque LL₂₀ et LL₄₀, respectivement pour des valeurs de 20 % et de 40 % de la valeur maximale d'activation d'un potentiel défini. Ces valeurs sont données à titre d'exemple, des valeurs différentes pourraient être utilisées dans des cas spécifiques.

### Test préalable de détermination de la phase compensatrice optimale

On va maintenant décrire plusieurs techniques algorithmiques permettant, selon l'invention, de déterminer les paramètres d'amplitude et/ou de durée de l'impulsion de précharge produite par le générateur du dispositif implanté, pour minimiser voire supprimer les effets secondaires indésirables produits par la phase compensatrice de la stimulation multiphasique.

La Figure 14 représente des caractéristiques isocharge pour différentes valeurs de charge (C=0,5 µC, 1 µC ou 1,5 µC) produites par une phase de compensation de la neurostimulation multiphasique, en superposition avec la caractéristique LL de la loi de Lapicque correspondant à la cible voulue pour l'effet physiologique recherché selon le cas, (cible "tout ou rien" ou bien cible quantifiée en pourcentage comme décrit plus haut dans les exemples d'application cliniques).

L'intersection X1, X2 ou X3 de ces deux courbes (isocharge et loi de Lapicque) correspond à un "point pivot" qu'il convient de déterminer pour ajuster les caractéristiques de la phase compensatrice, en particulier de l'impulsion de précharge, comme cela a été expliqué plus haut en relation avec la Figure 9.

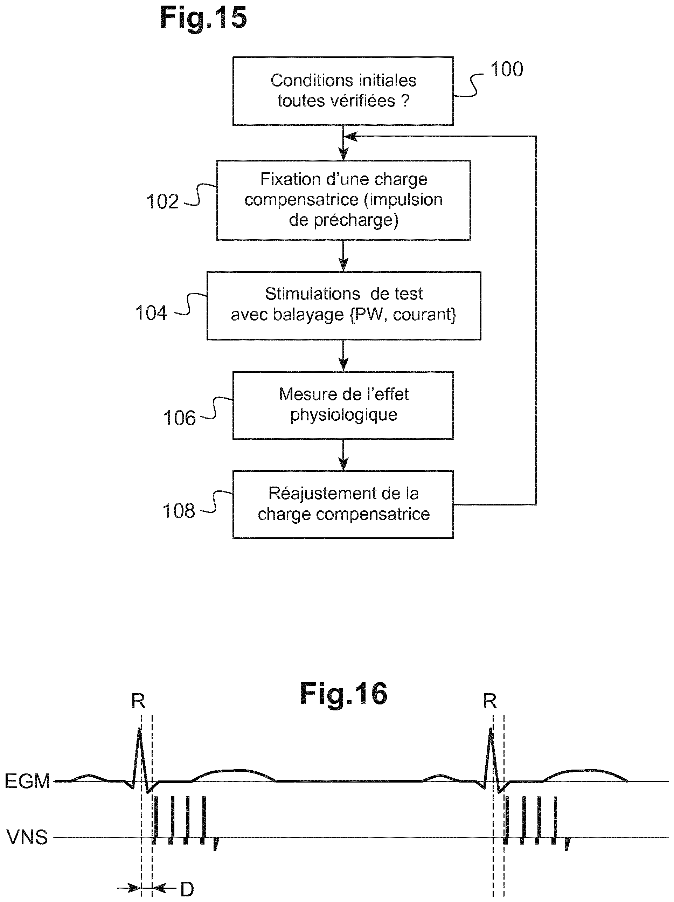

La Figure 15 illustre l'organigramme général des principales étapes de mise en oeuvre du test permettant de déterminer la charge de la phase compensatrice C_{pc} en fonction de la charge Cₐₚ de stimulation appliquée (dont les paramètres de courant et de longueur d'impulsion sont prédéterminés, qu'ils soient fixés par le médecin ou calculés par un algorithme spécifique).

La première étape (bloc 100) consiste à vérifier que les conditions initiales de mise en oeuvre de l'algorithme sont toutes vérifiées. Ces conditions peuvent être notamment :
- plage temporelle prédéterminée (par exemple une plage horaire choisie pour être toujours la même) ;
- patient au repos depuis un temps minimal prédéterminé (condition vérifiée à partir du signal produit par un capteur d'activité tel qu'un accéléromètre intégré au dispositif implanté) ;
- absence d'application d'une thérapie depuis un temps prédéterminé ;
- rythme cardiaque inférieur à la limite maximale ;
- rythme régulier, notamment absence de phase d'accélération ou de ralentissement des intervalles RR.

L'étape suivante (bloc 102) consiste à fixer un niveau donné de charge compensatrice, qui peut être un niveau égal à la charge de l'impulsion de stimulation ou bien qui peut également dépendre d'autres paramètres tels que le rythme cardiaque, la configuration d'électrodes, etc.

Le dispositif peut notamment déterminer, à partir du niveau de charge ainsi fixé, une série de couples de valeurs {largeur d'impulsion, courant} correspondant à cette valeur de charge. Par exemple, pour une charge prédéterminée C_{pc} = 1 µC, on définit le tableau suivant de valeurs de largeur d'impulsion avec leur niveau de courant associé.

**Tableau I**

| Index n° | PW (µs) | courant(mA) |
|---|---|---|
| 1 | 500 | 2.00 |
| 2 | 600 | 1.67 |
| 3 | 700 | 1.43 |
| 4 | 800 | 1.25 |
| 5 | 900 | 1.11 |
| 6 | 1000 | 100 |
| 7 | 1100 | 0.91 |
| 8 | 1200 | 0.83 |
| 9 | 1.300 | 0.77 |
| 10 | 1400 | 0.71 |
| 11 | 1500 | 0.67 |
| 17 | 1.600 | 0.63 |
| 13 | 1700 | 0.59 |
| 14 | 1800 | 0.56 |
| 15 | 1900 | 0.53 |
| 16 | 2000 | 0.50 |
| 17 | 2100 | 0.48 |
| 18 | 2200 | 0.45 |
| 19 | 2300 | 0.43 |
| 20 | 2400 | 0.42 |
| 21 | 2500 | 0.40 |
| 22 | 2600 | 0.38 |
| 23 | 2700 | 0.37 |
| 24 | 2800 | 0.36 |
| 25 | 2900 | 0.34 |
| 26 | 3000 | 0.33 |
| 27 | 3100 | 0.32 |
| 28 | 3200 | 0.31 |
| 29 | 3300 | 0.30 |
| 30 | 3400 | 0.29 |
| 31 | 3500 | 0.29 |
| 32 | 3600 | 0.28 |

L'étape suivante (bloc 104) consiste à appliquer une séquence de neurostimulation de test par un balayage des différentes valeurs possibles {largeur d'impulsion, courant} par des stimulations répétées, avec mesure dans chaque cas du paramètre physiologique et/ou physique susceptible d'être influencé par la délivrance de l'impulsion (bloc 106). La valeur de charge qui avait été établie à l'étape 102 est alors si besoin réajustée en fin de balayage (bloc 108), le processus précédent étant alors réitéré. Dans une mise en oeuvre, le système ne comporte qu'un système d'électrodes bipolaires ou quasi-tripolaires. Dans une autre mise en oeuvre, il comporte plusieurs électrodes permettant une stimulation sur plusieurs dipôles ou configurations d'électrodes. L'algorithme est alors appliqué à chaque système d'électrodes.

Dans une mise en oeuvre, l'algorithme de la Figure 15 est appliqué automatiquement par le stimulateur sans intervention extérieure. Dans une autre mise en oeuvre, il est appliqué sous le contrôle du médecin, au travers du système de communication, et permet de fixer les valeurs des impulsions de stimulation et des impulsions compensatrices. Dans une autre mise en oeuvre, une partie des fonctions sont réalisées par le stimulateur et une autre partie par le médecin au travers du système de communication.

Les Figures 16 à 18 illustrent de façon plus précise la manière dont sont appliqués les trains impulsionnels multiphasiques de cette séquence de test.

La Figure 16 montre l'application de deux trains impulsionnels multiphasiques de neurostimulation VNS délivrés en synchronisme avec le rythme cardiaque EGM. En effet, les impulsions peuvent être délivrées de manière asynchrone à la structure parasympathique mais, de préférence, elles sont délivrées de manière synchronisée sur les évènements cardiaques, comme illustré sur la Figure 16 : le train impulsionnel de neurostimulation est alors délivré de manière synchronisée sur l'onde P ou R, avec un délai D entre la détection de l'onde P ou R et le début du train impulsionnel de neurostimulation.

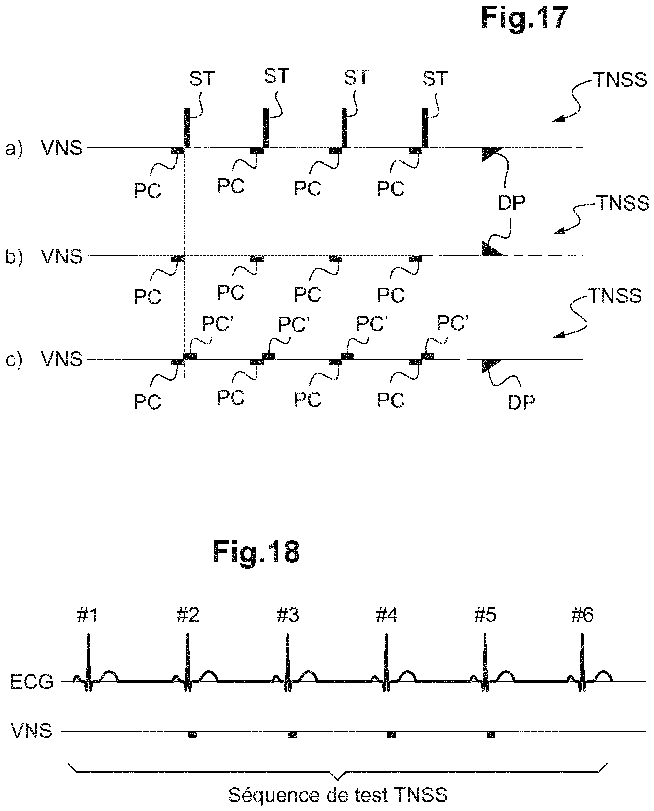

La Figure 17 illustre l'application d'un train impulsionnel pour une séquence de test TNSS où les trains impulsionnels appliqués présentent différents motifs de phases compensatrices :
- en a), la séquence de test comprend un nombre prédéterminé d'impulsions de précharge PC associées à autant d'impulsions de stimulation ST. Par exemple, la séquence de test TNSS comprend quatre phases de stimulation précédées chacune d'une impulsion de précharge ;
- dans la variante illustrée en b), seules des impulsions de précharge sont appliquées dans la séquence de test TNSS ;
- dans une autre variante encore, illustrée en c), l'impulsion de stimulation est remplacée par une impulsion PC' identique à l'impulsion de précharge PC mais de polarité inversée, de manière à éviter à la fin de la séquence de test TNSS que des charges électriques ne s'accumulent à l'interface entre l'électrode et les tissus physiologiques.

Comme illustré en Figure 18, la séquence de test TNSS complète comprend une succession de salves de stimulation (comme illustré en Figure 17) appliquée à des événements cardiaques consécutifs, par exemple les cycles cardiaques n° 2 à n° 5, cette séquence de test étant précédée (cycle n° 1) et suivies (cycle n° 6) par des cycles cardiaques sans neurostimulation.

### Recherche itérative des paramètres optimaux de la phase compensatrice

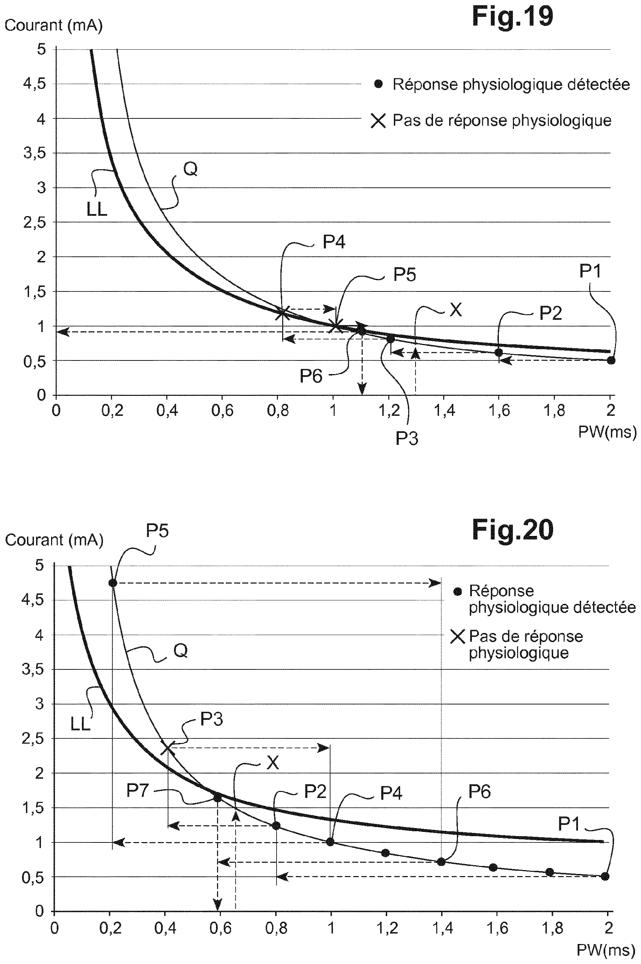

On va maintenant décrire, en référence aux Figures 19 à 26, plusieurs techniques algorithmiques permettant de réaliser le balayage des couples {largeur d'impulsion, courant} à l'étape 104 pour la recherche en un minimum de temps du point pivot X permettant de déterminer les meilleurs paramètres d'amplitude et/ou de durée à donner à l'impulsion de précharge, pour minimiser voire supprimer les effets secondaires indésirables produits par la phase compensatrice de la stimulation multiphasique.

La Figure 19 illustre une *première technique* de recherche du point optimal, basée sur un algorithme de type itérations dichotomiques, et la Figure 20 illustre une *deuxième technique,* basée sur un algorithme de type pseudo-stochastique (c'est-à-dire stochastique en excluant les points déjà testés).

Il s'agit dans l'un ou l'autre cas d'évaluer la position du point pivot X avec le moins d'itérations possibles.

Ces figures illustrent une superposition de la caractéristique isochrone Q avec le diagramme de la loi Lapicque LL, les ronds correspondent à des points situés au-dessous de la courbe de Lapicque LL, c'est-à-dire pour lesquels aucune réponse physiologique n'a été détectée, et les croix correspondent à des points situés au-dessus de cette même courbe, c'est-à-dire pour lesquels une réponse physiologique a été effectivement détectée.

Les différentes itérations sont désignées, dans l'ordre, par P₁, P₂, P₃... Dans l'algorithme dichotomique de la Figure 19, l'abscisse du point Pᵢ₊₁ est déterminée comme valant 90 % de l'abscisse du point Pi en l'absence de réponse physiologique attendue (c'est-à-dire si le point se trouve au-dessous de la courbe de Lapicque LL), l'abscisse de Pᵢ₊₁ étant telle que l'abscisse de Pᵢ soit de 80 % de celle de Pᵢ₊₁ dans le cas contraire. Dans l'algorithme pseudo-stochastique la Figure 20, les abscisses des points sont déterminées par un tirage aléatoire, en excluant les points déjà testés.

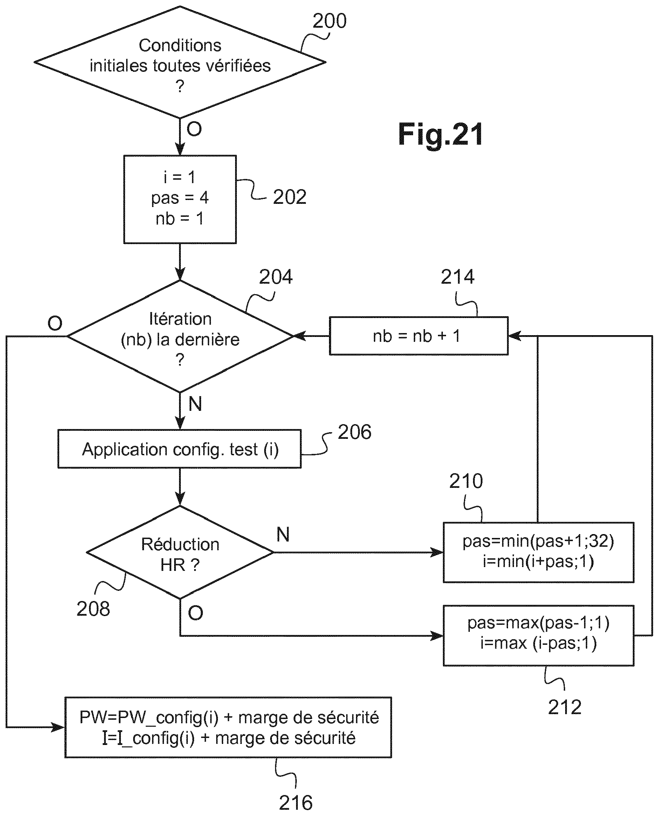

La Figure 21 illustre les différentes étapes d'un autre algorithme de recherche du point pivot, pour une *troisième technique* mettant en oeuvre une recherche de type "pas à pas". La teneur des étapes 200 à 216 et leur enchainement sont directement indiqués sur cette figure.

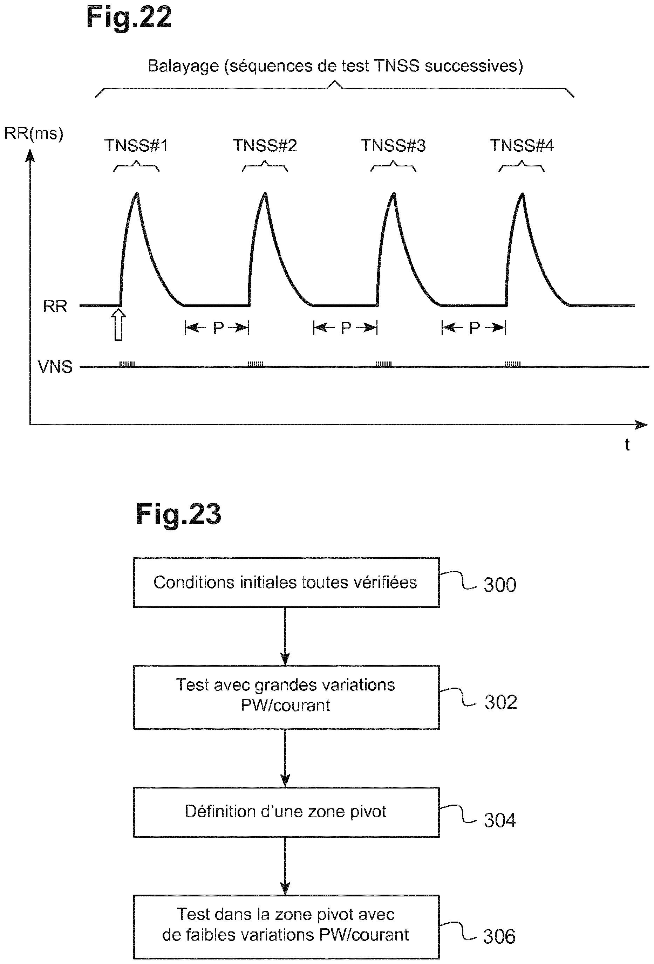

La Figure 22 illustre les variations du rythme cardiaque induites par des salves successives d'une séquence de test.

Quelle que soit la technique mise en oeuvre, pour chaque couple de valeurs {largeur d'impulsion, courant}, la séquence de test est appliquée une ou plusieurs fois, chaque séquence étant de préférence répétée un nombre prédéterminé de fois en prévoyant entre chaque séquence de test TNSS une période de "purge" P permettant le retour du paramètre physiologique testé (sur cette figure, les intervalles RR mesurant le rythme cardiaque de manière à éviter tout effet cumulatif entre les séquences de test successives).

Les Figures 23 à 25 illustrent une *quatrième technique* encore, consistant, dans un premier temps, à déterminer grossièrement sur la courbe {largeur d'impulsion, courant} une zone pivot encadrant le point pivot recherché et, dans un second temps, à rechercher ce point pivot par des variations fines restreintes au domaine de la zone pivot.

La Figure 23 illustre les étapes principales d'un tel algorithme.

Celui-ci débute (bloc 300) par une détermination de l'instant optimal pour effectuer le balayage, en vérifiant que les conditions initiales sont toutes vérifiées (comme à l'étape 100 de la Figure 15 ou à l'étape 200 de la Figure 21).

L'étape suivante (bloc 302) consiste à effectuer un premier test de balayage en parcourant la courbe largeur d'impulsion/courant avec des pas grands, par exemple des pas de 3 en 3 des valeurs d'index du Tableau 1 présenté plus haut.

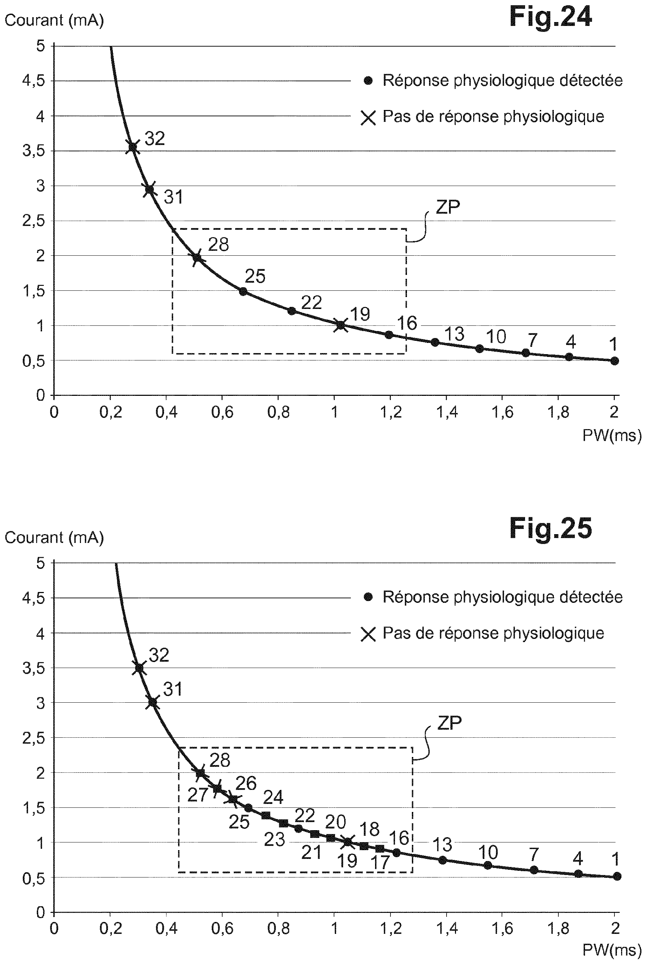

Ce balayage rapide est illustré sur la Figure 24, où l'on a indiqué les points correspondant à des valeurs d'index 1, 4, 7, 10, 13... du Tableau 1. Ce balayage peut être effectué par l'une des techniques décrites précédemment (dichotomique, pseudo-stochastique, pas à pas), ou toute autre méthode permettant de converger vers la zone pivot ZP). Dans l'exemple illustré, le premier balayage grossier est ainsi effectué avec seulement douze valeurs différentes de couples {largeur d'impulsion, courant}.

À partir des résultats obtenus (présence ou absence d'une réponse physiologique détectée pour chaque point de mesure), on définit la zone pivot ZP (bloc 304), par exemple à partir de la configuration la plus élevée et de la configuration la plus basse (en numéro d'index) ne produisant pas de changement de la réponse physiologique. Dans l'exemple illustré, la plus petite configuration est celle correspondant à l'index n° 16 (pas de changement de réponse entre les points 13 et 16) et la plus élevée est la configuration de l'index n° 28 (pas de modification de la réponse entre le point 28 et le point 31).

Le processus se poursuit alors (bloc 306) par mise en oeuvre de l'une des techniques décrites précédemment (recherche dichotomique, pseudo-stochastique, pas à pas ou autre), mais restreinte au domaine de la zone pivot ZP, comme illustré sur la Figure 25.

Cette recherche est effectuée avec un pas fin, d'une unité index, mais seulement sur une plage restreinte du Tableau 1. Dans l'exemple illustré en Figure 25, on avait déterminé à l'étape précédente que la zone pivot était bornée par les index n° 16 et n° 28, de sorte que la recherche fine sera opérée uniquement avec des couples {largeur d'impulsion, courant} compris entre l'index n° 17 et l'index n° 27.

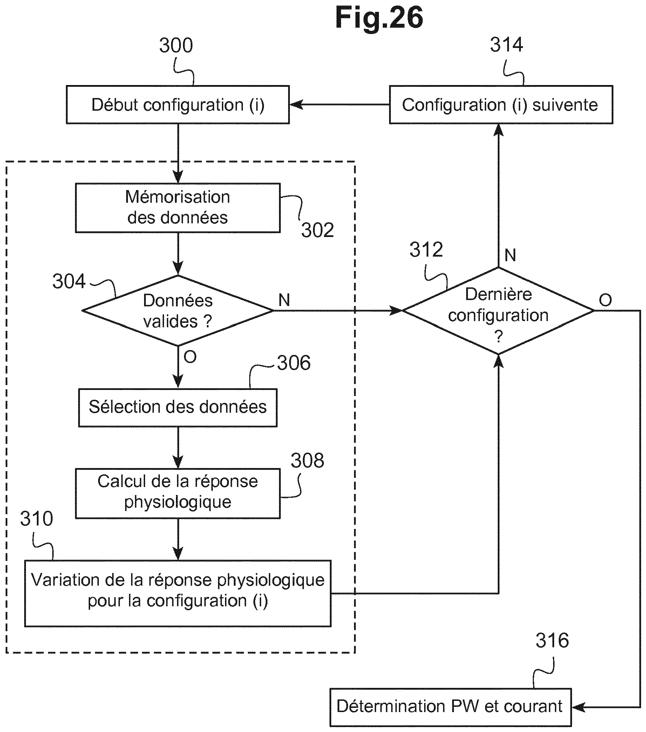

La Figure 26 est un organigramme général présentant les étapes successives de recherche de la configuration optimale, en fonction des variations de la réponse physiologique selon les diverses configurations.

L'organigramme commence à l'étape 300, pour une configuration i.

Les données produites par le ou les capteurs du paramètre physiologique et/ou physique reflétant la réponse physiologique sont mémorisés (bloc 302), avec éventuellement sélection parmi plusieurs données de celles qui sont les plus représentatives de la réponse physiologique à ce stade.

Les données sont ensuite validées (bloc 304) de manière à exclure celles qui sont, par exemple, obtenues dans les cas suivants :
- au moins un cycle cardiaque accéléré, par exemple une augmentation du paramètre HR de plus de 25 % d'un battement à l'autre ;
- au moins un cycle cardiaque décéléré, par exemple où le paramètre HR a diminué de plus de 25 % d'un cycle suivant ;
- patient en activité, par exemple dans le cas où le capteur d'accélération indique un niveau accru, supérieur à un seuil prédéterminé ;
- expiration d'une durée autorisée, par exemple une heure donnée, pour l'exécution de l'algorithme.

Si les données sont validées, on peut éventuellement opérer une sélection (bloc 306), par exemple en ne conservant que les x derniers cycles de la période de purge et les y derniers cycles de la période de stimulation. La réponse physiologique est ensuite calculée (bloc 308), par exemple par détermination d'une moyenne ou d'une médiane des données validées et sélectionnées.

Il est alors possible de déterminer (bloc 310) les variations de la réponse physiologique pour la configuration *i* en question.

Si cette configuration n'est pas la dernière (bloc 312), la configuration i+1 suivante est sélectionnée (bloc 314) et l'algorithme des blocs 300 à 310 est réitéré pour cette nouvelle configuration.

Une fois la dernière configuration atteinte, la largeur d'impulsion et le courant de la phase compensatrice sont déterminés (bloc 316), c'est-à-dire qu'un index du Tableau 1 est choisi, éventuellement avec application d'une marge de sécurité consistant à augmenter de deux unités cet index. Par exemple, si les valeurs de largeur d'impulsion et de courant limites sont obtenues pour l'index n° 5 (0,9 ms/1,11 mA) correspondant aux données les plus proches du point pivot X, alors les valeurs effectivement appliquées pour la largeur et le courant de l'impulsion de précharge seront celles correspondant à l'index n° 7 (1,1 ms/0,91 mA).

Dans une mise en oeuvre de l'invention, les paramètres sélectionnés sont automatiquement envoyés par le *Home Monitoring* à intervalles réguliers. Dans une autre mise en oeuvre, les paramètres ne sont envoyés que s'ils dépassent certaines valeurs limites considérées comme anormales.

Dans le cas d'une configuration multipolaire, plusieurs générateurs de courant sont utilisés. Chacun a potentiellement son propre profil de stimulation, comprenant des phases de stimulation et compensatrices.

Dans une première mise en oeuvre, chaque phase compensatrice de chaque générateur est gérée indépendamment des autres phases et générateurs, sur ses paramètres de durée et de courant.

Dans une seconde mise en oeuvre, les phases compensatrices sont gérées proportionnellement à l'injection de charges de leur phase de stimulation. Par exemple, si les phases compensatrices doivent être diminuées de 1 µC, la phase compensatrice d'un pôle délivrant 80 % du courant sera réduite de 0,8 µC (diminution de courant et/ou de durée).

## Revendications

1. Un dispositif médical actif (10), comprenant :
- un générateur (24) de neurostimulation électrique fonctionnelle (VNS), comprenant des moyens pour produire en sortie des trains impulsionnels multiphasiques de neurostimulation ;
- une sonde de stimulation (12), destinée à être disposée sur ou à proximité d'une structure (VN) du système nerveux d'un patient porteur du dispositif pour l'application à cette structure des trains impulsionnels de neurostimulation ;
- au moins un capteur (16) délivrant un signal de contrôle représentatif d'un paramètre physiologique et/ou physique susceptible d'être influencé par la délivrance des trains impulsionnels de neurostimulation ; et
- un circuit de contrôle automatique de compensation de charge (24, 26, 28), comprenant des moyens pour recevoir en entrée le signal de contrôle délivré par le au moins un capteur,
**caractérisé en ce que** chaque train impulsionnel comprend :
- au moins une impulsion de stimulation (ST) ; et
- au moins une impulsion compensatrice comprenant : une impulsion de précharge (PC) précédant l'impulsion de stimulation ; et/ou une impulsion de postcharge suivant l'impulsion de stimulation ; une impulsion de décharge passive (DP) terminant le train impulsionnel ; ou une combinaison des précédentes,
les impulsions de stimulation et compensatrice étant des impulsions de courant dont le sens, la durée (PW) et l'amplitude (I) sont contrôlés, et les impulsions compensatrices procurant une charge électrique cumulée compensant la charge électrique de la au moins une impulsion de stimulation,
et **en ce que** le circuit de contrôle automatique de compensation de charge (24, 26, 28) comprend en outre :
• des moyens pour déterminer une amplitude (I) et/ou une durée (PW) d'impulsion compensatrice en fonction d'au moins un critère prédéterminé ; et
• des moyens pour délivrer au générateur (24) un signal de pilotage des impulsions compensatrices à produire en sortie par celui-ci.

2. Le dispositif de la revendication 1, dans lequel le circuit de contrôle automatique de compensation de charge est un circuit comprenant :
• des moyens pour contrôler le générateur de manière à produire au moins un train impulsionnel de test (TNSS) ;
• des moyens pour recueillir le signal de contrôle après la fin du au moins un train impulsionnel de test ;
• des moyens pour déterminer d'après le niveau du signal de contrôle si un effet physiologique et/ou physique est ou non produit par l'application du train impulsionnel de test ; et
• des moyens pour déterminer l'amplitude et/ou la durée des impulsions compensatrices des trains impulsionnels suivants en fonction du résultat de la détermination.

3. Le dispositif de la revendication 2, dans lequel le train impulsionnel de test (TNSS) comprend une impulsion de précharge (PC) suivie d'au moins une impulsion de stimulation (ST).

4. Le dispositif de la revendication 3, dans lequel le circuit de contrôle automatique de compensation de charge comprend des moyens pour délivrer un unique train impulsionnel de test et des moyens pour recueillir le signal de contrôle immédiatement après la fin du train impulsionnel de test.

5. Le dispositif de la revendication 2, dans lequel le train impulsionnel de test comprend une impulsion de précharge (PC) mais ne comprend pas d'impulsion de stimulation (ST).

6. Le dispositif de la revendication 4, dans lequel le circuit de contrôle automatique de compensation de charge comprend des moyens pour délivrer une pluralité de trains impulsionnels de test successifs et des moyens pour recueillir le signal de contrôle après la fin du dernier train impulsionnel de test de ladite pluralité.

7. Le dispositif de la revendication 2, dans lequel le circuit de contrôle automatique de compensation de charge comprend des moyens pour déterminer l'amplitude et/ou la durée des impulsions compensatrices des trains impulsionnels courants en fonction de l'amplitude maximale de l'impulsion de décharge passive (DP) mesurée pour le train impulsionnel de test.

8. Le dispositif de la revendication 1, dans lequel le circuit de contrôle automatique de compensation de charge comprend en outre des moyens pour commander un intervalle temporel entre la fin de l'impulsion de stimulation (ST) et la fermeture d'interrupteurs (SW, SW') de mise au même potentiel des électrodes de stimulation, de manière à moduler l'instant de début de l'impulsion de décharge passive (DP).

9. Le dispositif de la revendication 1, dans lequel le circuit de contrôle automatique de compensation de charge comprend en outre des moyens pour commander un intervalle temporel entre la fin de l'impulsion de précharge (PC) et le début de l'impulsion de stimulation (ST).

10. Le dispositif de la revendication 1, dans lequel le circuit de contrôle automatique de compensation de charge comprend en outre des moyens pour commander un intervalle temporel entre la fermeture et l'ouverture d'interrupteurs (SW, SW') de mise au même potentiel des électrodes de stimulation, de manière à moduler la durée de l'impulsion de décharge passive (DP).

11. Le dispositif de la revendication 1, dans lequel ledit capteur est un capteur de mesure d'un paramètre du groupe formé par : activité électrique du coeur, activité électrique du système nerveux, rythme cardiaque, fréquence respiratoire, courant électrique traversant un tissu, accélération du corps du patient, pression sanguine, accélération endocardiaque, position du patient.

## Patentansprüche

1. Aktive medizinische Vorrichtung (10) umfassend:
- einen Generator (24) zur funktionalen elektrischen Nervenstimulation (VNS), umfassend Mittel zur Erzeugung und Ausgabe von mehrphasigen Impulsfolgen zur Nervenstimulation;
- eine Stimulationssonde (12) zur Anordnung auf oder in der Nähe einer Struktur (VN) des Nervensystems eines die Vorrichtung tragenden Patienten zum Anlegen der Impulsfolgen zur Nervenstimulation an diese Struktur;
- mindestens einen Sensor (16), der ein Steuerungssignal ausgibt, das für einen physiologischen und/oder physischen Parameter repräsentativ ist, der durch die Ausgabe der Impulsfolgen zur Nervenstimulation beeinflusst werden kann; und
- einen automatischen Steuerstromkreis zur Ladungskompensation (24, 26, 28), der Mittel zum Empfang des durch den mindestens einen Sensor ausgegebenen Steuersignals,
**dadurch gekennzeichnet, dass** jede Impulsfolge Folgendes umfasst:
- mindestens einen Stimulationsimpuls (ST); und
- mindestens einen Kompensationsimpuls umfassend: einen dem Stimulationsimpuls vorangehenden Vorladungsimpuls (PC); und/oder einen auf den Stimulationsimpuls folgenden Nachladungsimpuls; einen passiven Entladungsimpuls (DP), der die Impulsfolge abschließt; oder eine Kombination der vorhergehenden Impulse,
wobei die Stimulations- und Kompensationsimpulse Stromimpulse sind, deren Richtung, Dauer (PW) und Amplitude (I) gesteuert werden, und die Kompensationsimpulse eine gesamte elektrische Ladung bereitstellen, die die elektrische Ladung des mindestens einen Stimulationsimpuls kompensiert,
und dass der automatische Steuerstromkreis zur Ladungskompensation (24, 26, 28) außerdem Folgendes umfasst:
• Mittel zur Bestimmung einer Amplitude (I) und/oder einer Dauer (PW) des Kompensationsimpulses in Abhängigkeit mindestens eines vorbestimmten Kriteriums; und
• Mittel zur Übertragung an den Generator eines Steuersignals der zu erzeugenden Kompensationsimpulse.

2. Vorrichtung nach Anspruch 1, in der der automatische Steuerstromkreis zur Ladungskompensation ein Stromkreis ist, der Folgendes umfasst:
• Mittel zur Steuerung des Generators, um mindestens eine Test-Impulsfolge (TNSS) erzeugt;
• Mittel zur Erfassung des Steuersignals nach dem Ende der mindestens einen Test-Impulsfolge;
• Mittel zur Bestimmung, anhand der Stärke des Steuersignals, ob die Anwendung der Test-Impulsfolge eine physiologische und/oder physische Wirkung erzeugt oder nicht; und
• Mittel zur Bestimmung der Amplitude und/oder der Dauer der Kompensationsimpulse der folgenden Impulsfolgen in Abhängigkeit des Bestimmungsergebnisses.

3. Vorrichtung nach Anspruch 2, in der die Test-Impulsfolge (TNSS) einen Vorladungsimpuls (PC) gefolgt von mindestens einem Stimulationsimpuls umfasst.

4. Vorrichtung nach Anspruch 3, in der der automatische Steuerstromkreis zur Ladungskompensation Mittel zur Ausgabe einer einzigen Test-Impulsfolge sowie Mittel zur Erfassung des Steuersignals unmittelbar nach dem Ende der Test-Impulsfolge umfasst.

5. Vorrichtung nach Anspruch 2, in der die Test-Impulsfolge einen Vorladungsimpuls (PC) aber keinen Stimulationsimpuls (ST) umfasst.

6. Vorrichtung nach Anspruch 4, in der der automatische Steuerstromkreis zur Ladungskompensation Mittel zur Ausgabe einer Vielzahl von aufeinanderfolgenden Test-Impulsfolgen sowie Mittel zur Erfassung des Steuersignals unmittelbar nach dem Ende der letzten Test-Impulsfolge der Vielzahl umfasst.

7. Vorrichtung nach Anspruch 2, in der der automatische Steuerstromkreis zur Ladungskompensation Mittel zur Bestimmung der Amplitude und/oder der Dauer der Kompensationsimpulse der aktuellen Impulsfolgen in Abhängigkeit der maximalen Amplitude des für die Test-Impulsfolge gemessenen passiven Entladungsimpulses umfasst.

8. Vorrichtung nach Anspruch 1, in der der automatische Steuerstromkreis zur Ladungskompensation Mittel zur Steuerung eines Zeitintervalls zwischen dem Ende des Stimulationsimpulses (ST) und dem Ausschalten von Schaltern (SW, SW'), die dazu dienen, die Stimulationselektroden auf das gleiche elektrische Potenzial zu bringen, umfasst, um den Anfangszeitpunkt des passiven Entladungsimpulses anzupassen.

9. Vorrichtung nach Anspruch 1, in der der automatische Steuerstromkreis zur Ladungskompensation außerdem Mittel zur Steuerung eines Zeitintervalls zwischen dem Ende des Vorladungsimpulses (PC) und dem Anfang des Stimulationsimpulses (ST) umfasst.

10. Vorrichtung nach Anspruch 1, in der der automatische Steuerstromkreis zur Ladungskompensation ausserdem Mittel zur Steuerung eines Zeitintervalls zwischen dem Ausschalten und dem Einschalten von Schaltern (SW, SW'), die dazu dienen, die Stimulationselektroden auf das gleiche elektrische Potenzial zu bringen, umfasst, um die Dauer des passiven Entladungsimpulses anzupassen.

11. Vorrichtung nach Anspruch 1, in der der Sensor ein Sensor zur Messung eines Parameters ist, ausgewählt aus der Gruppe umfassend : die elektrische Aktivität des Herzens, die elektrische Aktivität des Nervensystems, den Herzrhythmus, die Atemfrequenz, einen ein Gewebe durchströmenden elektrischen Strom, eine Beschleunigung des Körpers des Patienten, den Blutdruck, eine endokardiale Beschleunigung, eine Position des Patienten.

## Claims

1. An active medical device (10) comprising:
- a generator (24) for functional electric nerve stimulation (VNS), comprising means for outputting multiphasic nerve stimulation pulse trains;
- a stimulation lead (12) to be disposed on or near a structure (VN) of the nervous system of a patient carrying the device for the application of the nerve stimulation pulse trains to this structure;
- at least one sensor (16) delivering a control signal representative of a physiological and/or physical parameter likely to be influenced by the delivery of the nerve stimulation pulse trains; and
- an automatic load compensation control circuit (24, 26, 28) including means for inputting the control signal from the at least one sensor,
**characterized in that** each pulse train comprises:
- at least one stimulation pulse (ST); and
- at least one compensating pulse comprising: a precharge pulse (PC) preceding the stimulation pulse; and/or an afterload pulse following the stimulation pulse; a passive discharge pulse (DP) terminating the pulse train; or a combination of the preceding,
the stimulation and compensating pulses being current pulses whose direction, duration (PW) and amplitude (I) are controlled, and the compensating pulses providing an accumulated electric charge compensating for the electrical charge of at least one stimulation pulse,
and **in that** the automatic load compensation control circuit (24, 26, 28) further comprises:
• means for determining a magnitude (I) and/or duration (PW) of the compensating pulse based on at least one predetermined criterion; and
• means for outputting to the generator (24) a drive signal of the compensating pulse to be generated by it.

2. The device according to claim 1, wherein the automatic load compensation control circuit is a circuit comprising:
• means for controlling the generator so as to produce at least one test pulse train (TNSS);
• means for receiving the control signal after the end of the at least one test pulse train;
• means for determining if a physiological and/or physical effect is produced or not by the application of the test pulse train, based on the level of the control signal; and
• means for determining the amplitude and/or duration of compensating pulses of the following pulse trains based on the determination results.

3. The device according to claim 2, wherein the test pulse train (TNSS) comprises a precharge pulse (CP) followed by at least one stimulation pulse (ST).

4. The device according to claim 3, wherein the automatic load compensation control circuit comprises means for delivering a single test pulse train and means for receiving the test signal immediately after the end of the test pulse train.

5. The device according to claim 2, wherein the test pulse train includes a precharge pulse (PC) but does not include a stimulation pulse (ST).

6. The device according to claim 4, wherein the automatic load compensation control circuit comprises means for delivering a plurality of successive test pulse trains and means for collecting the control signal after the end of the last test pulse train of said plurality.

7. The device according to claim 2, wherein the automatic load compensation control circuit comprises means for determining the amplitude and/or duration of compensating pulses of current pulse trains depending upon the maximum amplitude of the passive discharge pulse (DP) measured for the test pulse train.

8. The device according to claim 1, wherein the automatic load compensation control circuit further comprises means for controlling a time interval between the end of the stimulation pulse (ST) and closure of switches (SW , SW) for setting the stimulation electrodes to the same potential, so as to modulate the time of start of the passive discharge pulse (DP).

9. The device according to claim 1, wherein the automatic load compensation control circuit further comprises means for controlling a time interval between the end of the precharge pulse (PC) and the beginning of the pulse stimulation (ST).

10. The device according to claim 1, wherein the automatic load compensation control circuit further comprises means for controlling a time interval between the closing and opening of switches (SW, SW) for setting the stimulation electrodes to the same potential, so as to modulate the duration of the passive discharge pulse (DP).

11. The device according to claim 1, wherein said sensor is a sensor for measuring a parameter of the group consisting of: electrical activity of the heart, electrical activity of the nervous system, heart rate, respiratory frequency, electric current passing through a tissue, acceleration of the patient's body, blood pressure, endocardial acceleration, position of the patient.
